# EUROPEAN PATENT SPECIFICATION

(11) **EP 3 155 132 B1**
(45) Date of publication and mention of the grant of the patent: **01.08.2018**
(21) Application number: 16722957.4
(22) Date of filing: 25.02.2016
(51) Int. Cl.: C12Q 1/68

(54) **MARKERS FOR THE DETECTION OF BREVIBACILLUS LATEROSPORUS AND RELATED METHODS AND KITS**
MARKER FÜR DEN NACHWEIS VON BREVIBACILLUS LATEROSPORUS UND DAMIT VERBUNDENE VERFAHREN UND KITS
MARQUEURS POUR LA DÉTECTION DE BREVIBACILLUS LATEROSPORUS ET MÉTHODES ET TROUSSES ASSOCIÉES

(30) Priority: 26.02.2015 IT RM20150084
(43) Date of publication of application: 19.04.2017
(73) Proprietor: UNIVERSITA' DEGLI STUDI DI SASSARI, 07100 Sassari (IT)
(72) Inventor: RUIU, Luca, 07100 Sassari (SS) (IT); FALCHI, Giovanni, 07100 Sassari (SS) (IT); MARCHE, Maria Giovanna, 07100 Sassari (SS) (IT); MURA, Maria Elena, 07100 Sassari (SS) (IT)
(74) Representative: Gitto, Serena
(86) International application number: PCT/IT2016/000047
(87) International publication number: WO 2016/135766

(56) References cited:
- EP-A1- 1 788 093
- CN-A- 101 956 018
- DATABASE EMBL [Online] ebi; 16 March 2007 (2007-03-16), Rush: "genomic clone", XP002741920, Database accession no. ek400976
- LEI MENGYING ET AL: "Complete Genome Sequence of Paenibacillus polymyxa CF05, a Strain of Plant Growth-Promoting Rhizobacterium with Elicitation of Induced Systemic Resistance.", GENOME ANNOUNCEMENTS 2015, vol. 3, no. 2, 2015, XP002741921, ISSN: 2169-8287
- DJUKIC MARVIN ET AL: "Genome sequence of Brevibacillus laterosporus LMG 15441, a pathogen of invertebrates.", JOURNAL OF BACTERIOLOGY OCT 2011, vol. 193, no. 19, October 2011 (2011-10), pages 5535-5536, XP002741922, ISSN: 1098-5530
- DATABASE EMBL [Online] EBI; 29 October 2014 (2014-10-29), "Uncaracterised protein", XP002741923, Database accession no. A0A075R599
- DATABASE EMBL [Online] EBI; 22 February 2012 (2012-02-22), "Putative uncharacterised protein", XP002741924, Database accession no. H0UH74
- DE OLIVEIRA EDMAR JUSTO ET AL: "Molecular characterization of Brevibacillus laterosporus and its potential use in biological control.", APPLIED AND ENVIRONMENTAL MICROBIOLOGY NOV 2004, vol. 70, no. 11, November 2004 (2004-11), pages 6657-6664, XP002741925, ISSN: 0099-2240

## Description

The present invention concerns markers for the detection of *Brevibacillus laterosporus* and related methods and kits. Particularly, the present invention concerns markers for the detection, in any matrix, of any *Brevibacillus laterosporus* strains and for the detection of specific *Brevibacillus laterosporus* strains.

*Brevibacillus laterosporus* Laubach is a rod-shaped, endospore-forming bacterium morphologically characterized by the production of a typical canoe-shaped parasporal body (CSPB) firmly attached to one side of the spore, which determines its lateral position in the sporangium. It is an ubiquitous species that has been isolated from a wide range of materials including soil, gemstones, lahar, fresh water, sea water, insect bodies, leaf surfaces, locust beans, compost, milk, cheese, honey, starchy foods, gelatin-factory effluents, animal hide and wool, quails (Ruiu et al., 2013). This bacterium is considered non pathogen to humans and due to the specific properties of different strains it finds applications in diverse industrial sectors. This includes its use as a biopesticide (i.e. insecticide, fungicide, nematicide and moluscicide), a biofertilizers, in the bioremediation for pollution management, in the biomedical sectors for the production of antibiotics.

The interest on this bacterial species is raising, so that there is a need to implement methods for its detection and traceability in the environment and in the industrial production processes.

Several methods for detecting *Brevibacillus laterosporus* are known (EP1788093 and CN101956018) wherein oligonucleotide sequences corresponding to a 16S ribosomial RNA gene of bacteria are targeted for detection. However, 16S ribosomial RNA gene is present in a large number of bacteria with high homology level. Therefore, a detection based on this gene shows a low efficiency. In addition, the expression level of 16S ribosomial RNA gene represents a limit to the sensibility of the detection method.

In the light of above it is therefore apparent the need to provide for new methods able to overcome the disadvantages of the known methods for detecting *Brevibacillus laterosporus.*

Therefore, the present invention has the aim to provide a method for the detection of the bacterial species *Brevibacillus laterosporus* able to specifically detect the presence of *B. laterosporus* and to quantify it in different matrices even if the bacterial species was present in low amount.

More in detail the invention deals with a PCR-based method, preferably RT-PCR method, for the detection of the bacterium *Brevibacillus laterosporus* employing nucleotide sequences targeting specific genes of this species.

Target genes have been identified through a study combining a proteomic and genomic approach that highlighted the expression of these genes and the localization of proteins in the bacterial cell.

These genes encodes for proteins that can be extracted from the surface of spores contained in spore suspensions obtained through culture in different solid or liquid media (i.e. Luria Bertani Broth) with methods known in the art (Ruiu et al., 2007). Typically the surface of these spores is covered by a complex including the spore coat and the canoe shaped parasporal body (SC-CSPB-complex) that is a unique feature of this bacterial species (Fitz-James and Young, 1958). The proteins of the above mentioned complex can be extracted by alkali, but they had never been identified prior to the present invention.

Among other proteins, two proteins were identified as corresponding to proteins whose localization in the bacterial cell and function was unknown. The previous knowledge of the sequences of the genes encoding for these two proteins is the result of the whole or partial genome sequencing of strains LMG 15441, GI-9, PE36, B9, and DSM 25. The sequences and accession numbers of these genes in different strains of *Brevibacillus laterosporus* are shown in Examples 1 and 2. The first gene, having sequence SEQ ID NO:1 in *Brevibacillus laterosporus* LMG 15441, encodes for a protein with a molecular weight around 28 KDa, while the second gene, having sequence SEQ ID NO:2 in *Brevibacillus laterosporus* GI-9, encodes for a protein with a molecular weight around 14 kDa.

For the first time, the actual expression of these genes has been verified and the encoded proteins have been associated to the SC-CSPB complex. In addition, the inventors have discovered that alike the SC-CSPB complex, these two genes are typical of *Brevibacillus laterosporus* and are not found in other bacterial species.

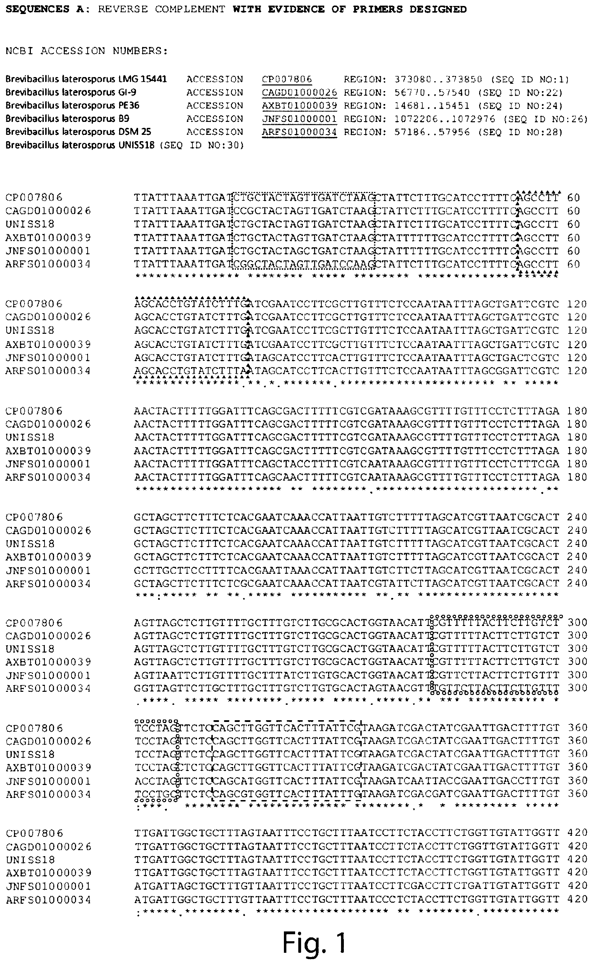

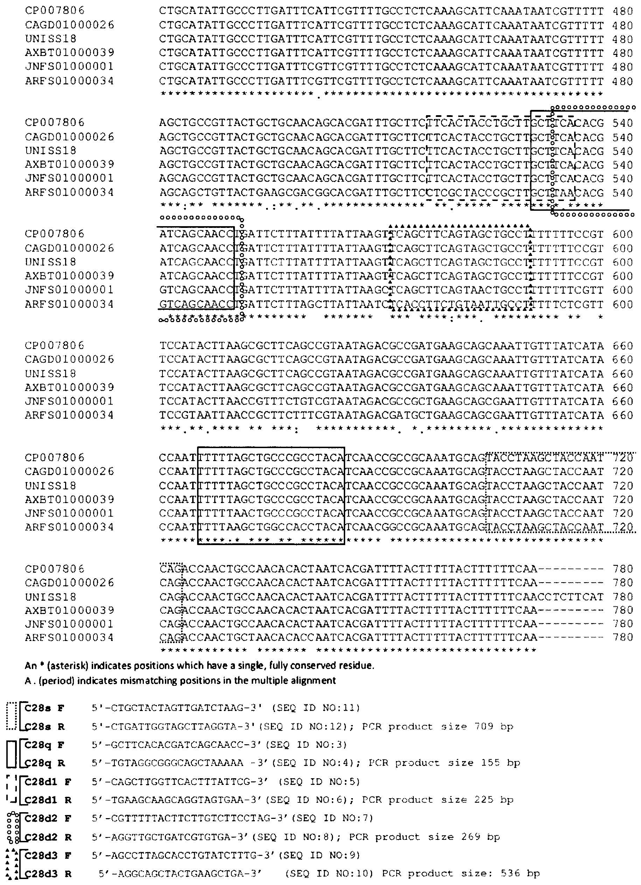

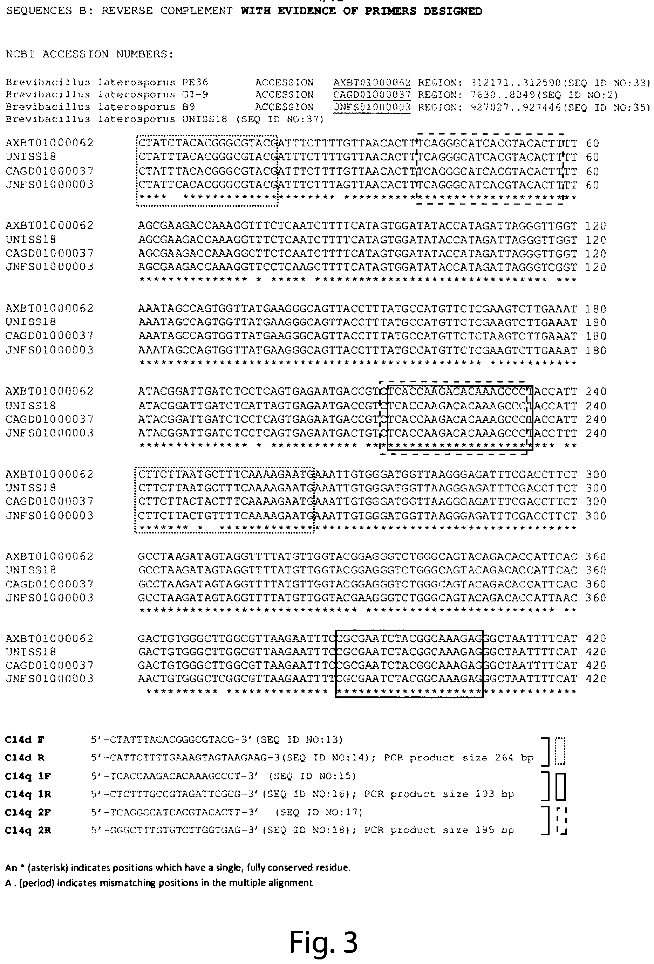

The above mentioned sequences show an high identity among the different *Brevibacillus laterosporus,* as shown in figure 1 and 3. However, since slight differences exist among the same sequence of different strains, the nucleotide sequences encoding for a protein associated to the SC-CSPB complex and having a molecular weight around 28 KDa of different *Brevibacillus laterosporus* strains is hereafter reported as sequence A, whereas the nucleotide sequences encoding for a protein associated to the SC-CSPB complex and having a molecular weight around 14 KDa of different *Brevibacillus laterosporus* strains is hereafter reported as sequence B.

Consequently, pairs of primers designed on the nucleotide sequences of the above mentioned two genes can be used for the detection of any *Brevibacillus laterosporus* strains or, alternatively, for the detection of specific *Brevibacillus laterosporus* strains, in different matrices. More in detail, the first gene, having sequence SEQ ID NO:1 in *Brevibacillus laterosporus* LMG 15441 and encoding for a protein with a molecular weight around 28 KDa, can be used for the detection of any *Brevibacillus laterosporus* strain, while the second gene, having sequence SEQ ID NO:2 in *Brevibacillus laterosporus* GI-9 and encoding for a protein with a molecular weight around 14 kDa, can be used for the detection of specific *Brevibacillus laterosporus* strains with special reference to strain UNISS 18 deposited with NCIMB N° 41419 in the NCIMB Ltd. Aberdeen, UK and patented for the biological control of dipters (European Patent No 2,079,314; United States Patent No 8,076,119).

Any primer pairs (oligonucleotide) designed on nucleotide sequences A and B can specifically bind and amplify part or the complete genes with sequences A and B in PCR reactions if *B. laterosporus* genome is present as a template. As a proof of the specificity of this detection technique, if the genome of other bacterial species is used as a template, no amplification of the expected PCR product is obtained.

In addition, the detection method according to the present invention is able to detect *B. laterosporus* even if it was present in low amount in a sample. As showed in the examples, in fact, the inventors have found that the expression level of sequence A is high and higher than 16S ribosomial RNA gene used in the known detection methods.

The method of the invention is therefore more specific and sensitive in comparison to known methods for detecting *B. laterosporus.*

The present disclosure describes a marker for use in the detection and/or quantification of *Brevibacillus laterosporus* in a sample, said marker consisting of a nucleic acid sequence encoding a surface polypeptide of the spore coat and the canoe shaped parasporal body of *Brevibacillus laterosporus,* said nucleic acid sequence comprising or consisting of:
a) SEQ ID NO: 1;
b) SEQ ID NO: 2;
c) a fragment of the nucleic acid sequences a) or b), said fragment having at least 12 bp, preferably from 15 to 30 bp, more preferably from 18 to 24 bp; or
d) a nucleic acid sequence having a sequence identity of at least 80%, preferably 90% with any of the nucleic acid sequences a)-c);
e) a complement nucleic sequence of said sequences a)-d)

The above mentioned identity can be determined by Basic Local Alignment search Tool (BLAST) of the National Center for Biotechnology Information (NCBI).

The nucleic acid sequences having a sequence identity of at least 80% with any of the nucleic acid sequences a)-c) are the nucleic acid sequences of different *Brevibacillus laterosporus* strains.

In addition, the present disclosure describes a marker for use in the detection and/or quantification of *Brevibacillus laterosporus* in a sample, said marker consisting of a surface polypeptide sequence of the spore coat and the canoe shaped parasporal body of *Brevibacillus laterosporus,* said surface polypeptide sequence comprising or consisting of:
f) SEQ ID NO: 19;
g) SEQ ID NO: 20;
h) a fragment of the polypeptide sequence f) or g) having at least 5 aminoacids, preferably from 6 to 20 aminoacids, more preferably from 8 to 15 aminoacids;
i) a polypeptide sequence having a sequence identity of at least 90% with any of the polypeptide sequences f)-h). The above mentioned identity can be determined by Basic Local Alignment search Tool (BLAST) of the National Center for Biotechnology Information (NCBI).

The polypeptide sequences having a sequence identity of at least 90% with any of the polypeptide sequences f)-h) are the polypeptide sequences of different *Brevibacillus laterosporus* strains.

According to the present invention, *Brevibacillus laterosporus* that can be detected and/or quantified are for example *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9* (Sharma et al., 2012), *Brevibacillus laterosporus PE36* (Theodore et al., 2014), *Brevibacillus laterosporus B9* (Li et al., 2014), *Brevibacillus laterosporus DSM25* (ATCC 64).

On the basis of the above, it is a specific object of the present invention a method for the detection and/or quantification of *Brevibacillus laterosporus* in a sample, said method comprising or consisting of the detection and/or quantification of at least one marker selected from the group consisting of:
a nucleic acid sequence comprising or consisting of SEQ ID NO:1 or the complement nucleic acid sequence thereof; a fragment of said SEQ ID NO:1 or complement nucleic acid sequence thereof, said fragment having at least 12 bp, preferably from 15 to 30 bp, more preferably from 18 to 24 bp; a nucleic acid sequence having a sequence identity of at least 80%, preferably 90% with said SEQ ID NO: 1, said complement nucleic acid sequence or said fragment;
a surface polypeptide sequence comprising or consisting of SEQ ID NO: 19, a fragment thereof having at least 5 aminoacids, preferably from 6 to 20 aminoacids, more preferably from 8 to 15 aminoacids;
a polypeptide sequence having a sequence identity of at least 90% with said SEQ ID NO: 19 or with said fragment thereof having at least 5 aminoacids, preferably from 6 to 20 aminoacids, more preferably from 8 to 15 aminoacids. For example, the peptide can be detected by ELISA or Western Blot methods.

As mentioned above, the identity of the sequences can be determined by Basic Local Alignment search Tool (BLAST) of the National Center for Biotechnology Information (NCBI).

According to the present invention, *Brevibacillus laterosporus* that can be detected and/or quantified are for example *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9* (Sharma et al., 2012), *Brevibacillus laterosporus PE36* (Theodore et al., 2014), *Brevibacillus laterosporus B9* (Li et al., 2014), *Brevibacillus laterosporus DSM25* (ATCC 64).

A further aspect of the present invention concerns a method for the detection and/or quantification in a sample of *Brevibacillus laterosporus,* said method comprising or consisting of the detection of at least one marker selected from the group consisting of:
a nucleic acid sequence comprising or consisting of: SEQ ID NO:2 or the complement nucleic acid sequence thereof; a fragment of said SEQ ID NO:2 or complement nucleic acid sequence thereof, said fragment having at least 12 bp, preferably from 15 to 30 bp, more preferably from 18 to 24 bp; or
a nucleic acid sequence having a sequence identity of at least 80%, preferably 90% with said SEQ ID NO: 2, the complement nucleic acid sequence or said fragment;
a surface polypeptide sequence comprising or consisting of: SEQ ID NO: 20, a fragment thereof having at least 5 aminoacids, preferably from 6 to 20 aminoacids, more preferably from 8 to 15 aminoacids;
a polypeptide sequence having a sequence identity of at least 90% with said SEQ ID NO: 20 or with said fragment thereof having at least 5 aminoacids, preferably from 6 to 20 aminoacids, more preferably from 8 to 15 aminoacids.

For example, this method can detect *Brevibacillus laterosporus* NCIMB 41419.

According to a specific embodiment of the present invention, the method of claims 1-2 can be carried out by means of PCR technique or Real Time PCR by the use of at least one of the following primer pairs:
5'-GCTTCACACGATCAGCAACC-3' (SEQ ID NO:3)
5'-TGTAGGCGGGCAGCTAAAAA -3' (SEQ ID NO:4);
5'-C AGC TTG GTT CAC TTT ATT CG-3' (SEQ ID NO:5)
5'-TG AAG CAA GCA GGT AGT GAA-3'(SEQ ID NO:6);
5'-CGT TTT TAC TTC TTG TCT TCC TAG-3'(SEQ ID NO:7)
5'-AG GTT GCT GAT CGT GTG A-3'(SEQ ID NO:8);
5'-AGC CTT AGC ACC TGT ATC TTT G-3'(SEQ ID NO:9)
5'-AG GCA GCT ACT GAA GCT GA-3'(SEQ ID NO:10);
5'-CTGCTACTAGTTGATCTAAG-3'(SEQ ID NO:11)
5'-CTGATTGGTAGCTTAGGTA-3'(SEQ ID NO:12);
preferably
5'-CTGCTACTAGTTGATCTAAG-3' (SEQ ID NO:11) 5'-CTGATTGGTAGCTTAGGTA-3'(SEQ ID NO:12) or
5'-GCTTCACACGATCAGCAACC-3' (SEQ ID NO:3) 5'-TGTAGGCGGGCAGCTAAAAA -3' (SEQ ID NO:4).

The primers SEQ ID NO:11 and 12 are preferable in the detection carried out by PCR, whereas primers SEQ ID NO: 3 and 4 are preferable in the detection carried out by Real Time PCR.

The method according to claims 3-4 can be carried out by means of PCR technique or by Real Time PCR by the use of at least one of the following primer pairs:
5'-CTA TTT ACA CGG GCG TAC G-3'(SEQ ID NO:13) 5'-CAT TCT TTT GAA AGTAGTAAG AAG-3'(SEQ ID NO:14);
5'-TCACCAAGACACAAAGCCCT-3'(SEQ ID NO:15)
5'-CTCTTTGCCGTAGATTCGCG-3'(SEQ ID NO:16);
5'-TCAGGGCATCACGTACACTT-3' (SEQ ID NO:17)
5'-GGGCTTTGTGTCTTGGTGAG-3'(SEQ ID NO:18);
preferably
5'-CTA TTT ACA CGG GCG TAC G-3'(SEQ ID NO:13)
5'-CAT TCT TTT GAA AGTAGTAAG AAG-3'(SEQ ID NO:14)

According to a further embodiment, the present invention concerns also a method for the detection of *Brevibacillus laterosporus* in a sample, said method comprising the method as defined in anyone of the claims 1-2, 5 and the method as defined in anyone of the claims 3-4, 6.

In addition, the present invention concerns a kit for the detection and/or quantification of *Brevibacillus laterosporus* in a sample, said kit comprising or consisting of at least one of the following primer pairs:
5'-GCTTCACACGATCAGCAACC-3' (SEQ ID NO:3)
5'- TGTAGGCGGGCAGCTAAAAA -3'(SEQ ID NO:4);
5'-C AGC TTG GTT CAC TTT ATT CG-3' (SEQ ID NO:5)
5'-TG AAG CAA GCA GGT AGT GAA-3'(SEQ ID NO:6);
5'-CGT TTT TAC TTC TTG TCT TCC TAG-3'(SEQ ID NO:7)
5'-AG GTT GCT GAT CGT GTG A-3'(SEQ ID NO:8);
5'-AGC CTT AGC ACC TGT ATC TTT G-3'(SEQ ID NO:9)
5'-AG GCA GCT ACT GAA GCT GA-3'(SEQ ID NO:10);
5'-CTGCTACTAGTTGATCTAAG-3'(SEQ ID NO:11)
5'-CTGATTGGTAGCTTAGGTA-3'(SEQ ID NO:12);
preferably
5'-CTGCTACTAGTTGATCTAAG-3' (SEQ ID NO:11)
5'-CTGATTGGTAGCTTAGGTA-3'(SEQ ID NO:12) - or
5'-GCTTCACACGATCAGCAACC-3' (SEQ ID NO:3)
5'-TGTAGGCGGGCAGCTAAAAA -3' (SEQ ID NO:4).
together with suitable reactive agents for the detection and/or quantification for instance by PCR and/or Real Time PCR.

The primers SEQ ID NO:11 and 12 are preferable in the detection carried out by PCR, whereas primers SEQ ID NO: 3 and 4 are preferable in the detection carried out by Real Time PCR.

The kit can be used for the detection and/or quantification of different *Brevibacillus laterosporus* strains chosen for example from the group consisting of *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus Gl-9* (Sharma et al., 2012), *Brevibacillus laterosporus PE36* (Theodore et al., 2014), *Brevibacillus laterosporus B9* (Li et al., 2014), *Brevibacillus laterosporus DSM25* (ATCC 64).

In addition, the present invention concerns a kit for the detection and/or quantification in a sample of *Brevibacillus laterosporus,* said kit comprising or consisting of at least one of the following primer pairs:
5'-CTA TTT ACA CGG GCG TAC G-3'(SEQ ID NO:13)
5'-CAT TCT TTT GAA AGTAGTAAG AAG-3'(SEQ ID NO:14);
5'-TCACCAAGACACAAAGCCCT-3'(SEQ ID NO:15)
5'-CTCTTTGCCGTAGATTCGCG-3'(SEQ ID NO:16);
5'-TCAGGGCATCACGTACACTT-3' (SEQ ID NO:17)
5'-GGGCTTTGTGTCTTGGTGAG-3'(SEQ ID NO:18);
preferably
5'-CTA TTT ACA CGG GCG TAC G-3'(SEQ ID NO:13)
5'-CAT TCT TTT GAA AGTAGTAAG AAG-3'(SEQ ID NO:14)
together with suitable reactive agents for the detection and/or quantification for example by PCR and/or Real Time PCR

Said kit can be used for detection and/or quantification for example of *Brevibacillus laterosporus* NCIMB 41419.

According to a further aspect of the present invention, the invention concerns a kit for the detection and/or quantification in a sample of *Brevibacillus laterosporus,* said kit comprising or consisting of the combination of the kit according to claims 8-9 and 10-11.

The present invention now will be described by illustrative but not limitative way according to preferred embodiment thereof with particular reference to the enclosed drawings, wherein:
**Figure 1** shows the alignment of the antisense sequences SEQ ID NO: 1, 22, 24, 26, 28 and 30 encoding for a protein associated to the SC-CSPB complex and having a molecular weight around 28 KDa in different *Brevibacillus laterosporus* strains.

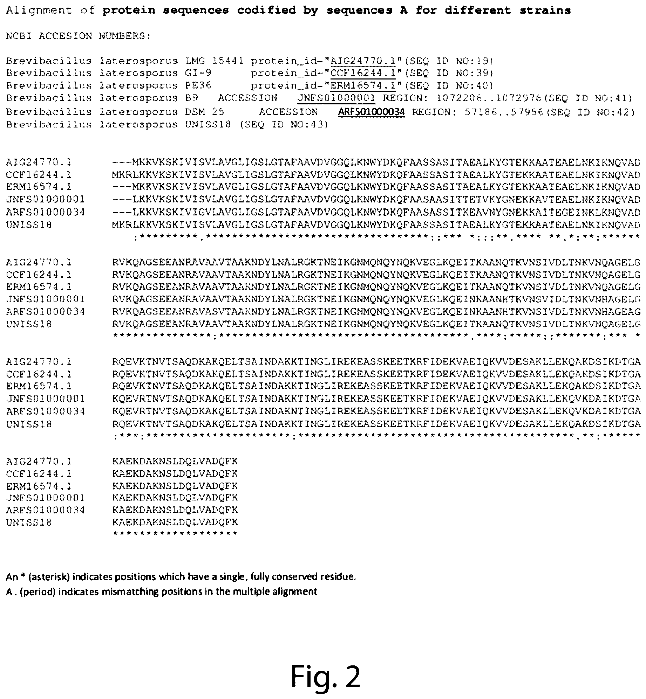

**Figure 2** shows the alignment of the polypeptide sequences SEQ ID NO: 19, 39, 40, 41, 42, 43 codified by the antisense sequences SEQ ID NO: 1, 22, 24, 26, 28 and 30 respectively.
**Figure 3** shows the alignment of the antisense sequences SEQ ID NO: 33, 2, 35, 37 encoding for a protein associated to the SC-CSPB complex and having a molecular weight around 14 kDa.

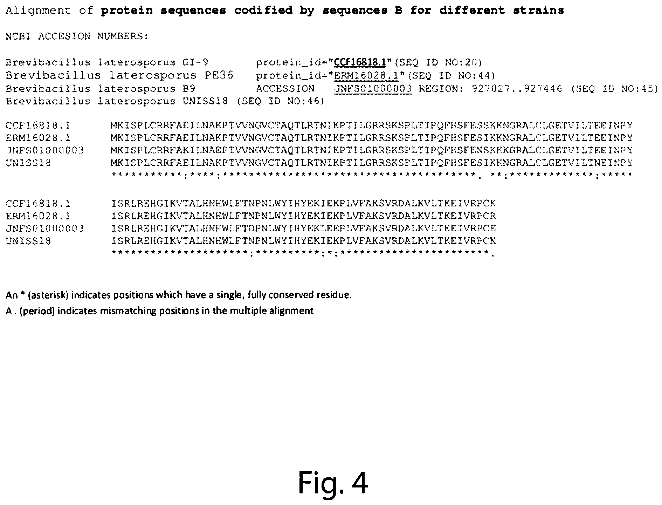

**Figure 4** shows the alignment of the polypeptide sequences SEQ ID NO: 44, 20, 45, 46 codified by the antisense sequences SEQ ID NO: 33, 2, 35, 37 respectively.

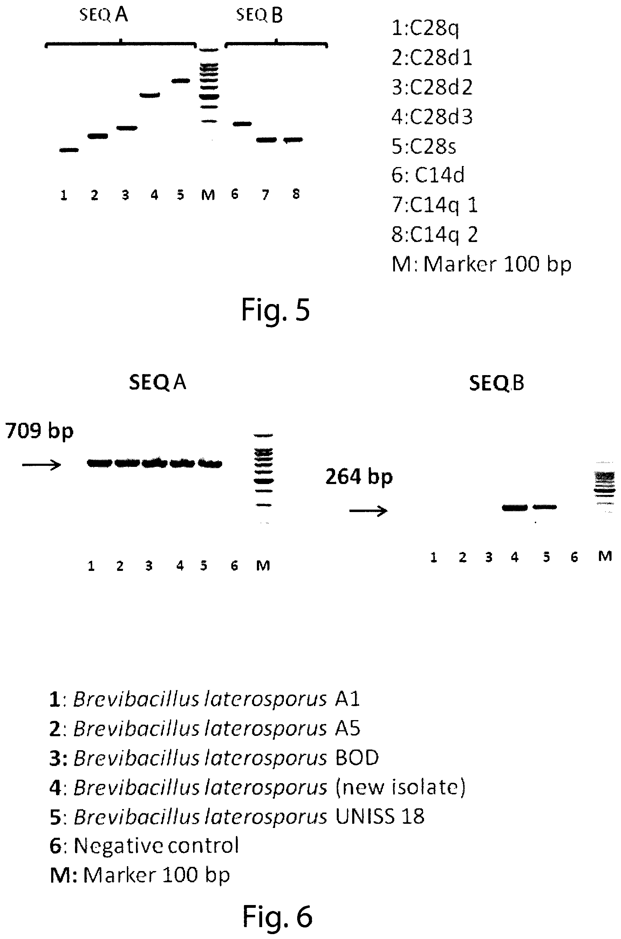

**Figure 5** shows the PCR results to detect SEQ ID NO: 30 and SEQ ID NO: 37 on *Brevibacillus laterosporus* UNISS 18. For SEQ ID NO: 30 the following primers pairs have been used: (1) C28q ; (2) C28d1; (3) C28d2; (4) C28d3; (5) C28s; and the PCR products size obtained were: (1) 155 bp; (2) 225 bp; (3) 269 bp; (4) 536 bp; (5) 709 bp. For SEQ ID NO: 37 the following primers pair have been used: (6) C14d ; (7) C14q1; (8) C14q2; and the PCR products size obtained were: (6) 264 bp; (7) 193 bp; (8) 195 bp.
**Figure 6** shows the PCR results to detect Sequences A and B on DNA extracted with commercial kit from different *Brevibacillus laterosporus* strains : (1) *Brevibacillus laterosporus* A1; (2) *Brevibacillus laterosporus* A5; (3) *Brevibacillus laterosporus* BOD; (4) *Brevibacillus laterosporus* (new isolate); (5) *Brevibacillus laterosporus* UNISS18; (6) Negative control; (M) Marker 100 bp. For Sequence A the PCR products of 709 bp were obtained using C28s primers pair; for Sequence B the PCR products of 264 bp were obtained using C14d primers pair.

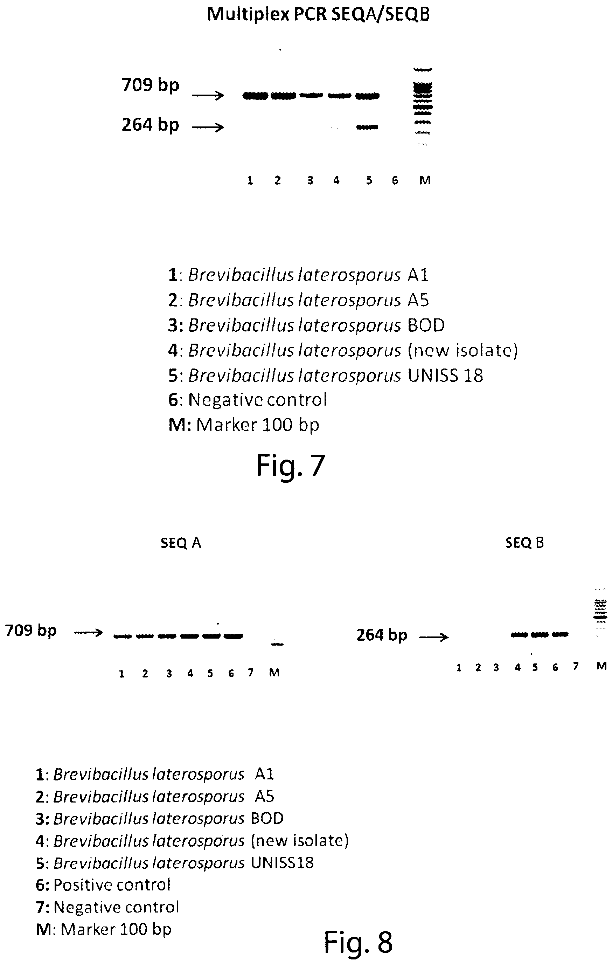

**Figure 7** shows the Multiplex PCR results to detect sequences A and B simultaneously on DNA extracted with commercial kit from different *Brevibacillus laterosporus* strains: (1) *Brevibacillus laterosporus* A1; (2) *Brevibacillus laterosporus* A5; (3) *Brevibacillus laterosporus* BOD; (4) *Brevibacillus laterosporus* (new isolate); (5) *Brevibacillus laterosporus* UNISS18; (6) Negative control; (M) Marker 100 bp. For Sequence A the PCR products of 709 bp were obtained using C28s primers pair; for Sequence B the PCR products of 264 bp were obtained using C14d primers pair.
**Figure 8** shows the PCR results to detect sequences A and B on DNA extracted with boiling method from different *Brevibacillus laterosporus* strains: (1) *Brevibacillus laterosporus* A1; (2) *Brevibacillus laterosporus* A5; (3) *Brevibacillus laterosporus* BOD; (4) *Brevibacillus laterosporus* (new isolate); (5) *Brevibacillus laterosporus* UNISS18; (6) Positive control; (7) Negative control; (M) Marker 100 bp. For sequence A the PCR products of 709 bp were obtained using C28s primers pair; for sequence B the PCR products of 264 bp were obtained using C14d primers pair.

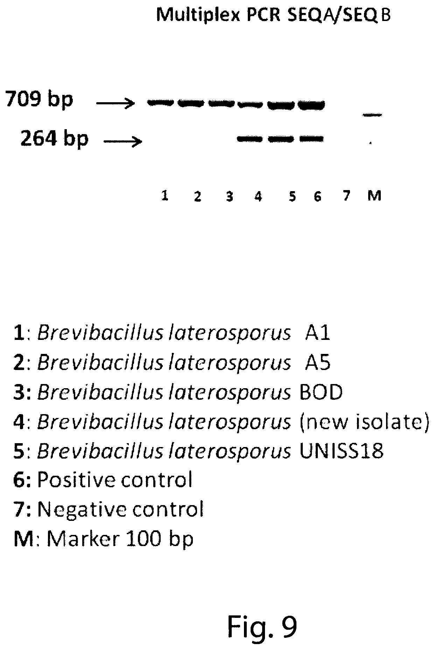

**Figure 9** shows the Multiplex PCR results to detect sequences A and B on DNA extracted with boiling method from different *Brevibacillus laterosporus* strains: (1) *Brevibacillus laterosporus* A1; (2) *Brevibacillus laterosporus* A5; (3) *Brevibacillus laterosporus* BOD; (4) *Brevibacillus laterosporus* (new isolate); (5) *Brevibacillus laterosporus* UNISS18; (6) Positive control; (7) Negative control; (M) Marker 100 bp. For sequence A the PCR products of 709 bp were obtained using C28s primers pair; for sequence B the PCR products of 264 bp were obtained using C14d primers pair.

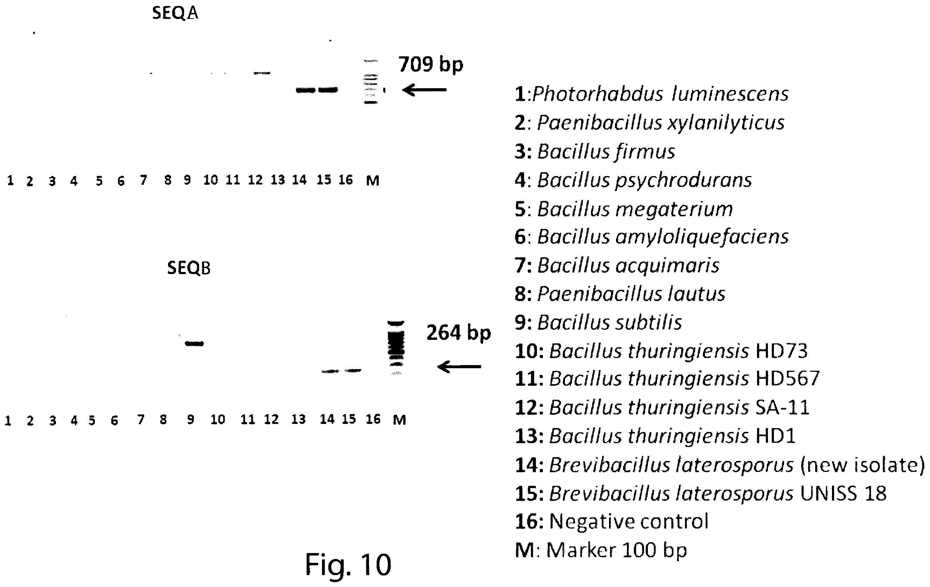

**Figure 10** shows the PCR results to detect sequences A and B on DNA extracted with commercial kit from different bacterial species : (1) *Photorhabdus luminescens,* (2) *Paenibacillus xylanilyticus,* (3) *Bacillus firmus,* (4) *Bacillus psychrodurans,* (5) *Bacillus megaterium,* (6) *Bacillus amyloliquefaciens,* (7) *Bacillus acquimaris,* (8) *Paenibacillus lautus,* (9) *Bacillus subtilis,* (10) *Bacillus thuringiensis* HD73, (11) *Bacillus thuringiensis* HD567, (12) *Bacillus thuringiensis* SA-11, (13) *Bacillus thuringiensis* HD1, (14) *Brevibacillus laterosporus* (new isolate), (15) *Brevibacillus laterosporus* UNISS 18 (16) Negative control; (M) Marker 100 bp. For sequence A the PCR products of 709 bp were obtained using C28s primers pair; for sequence B the PCR products of 264 bp were obtained using C14d primers pair.

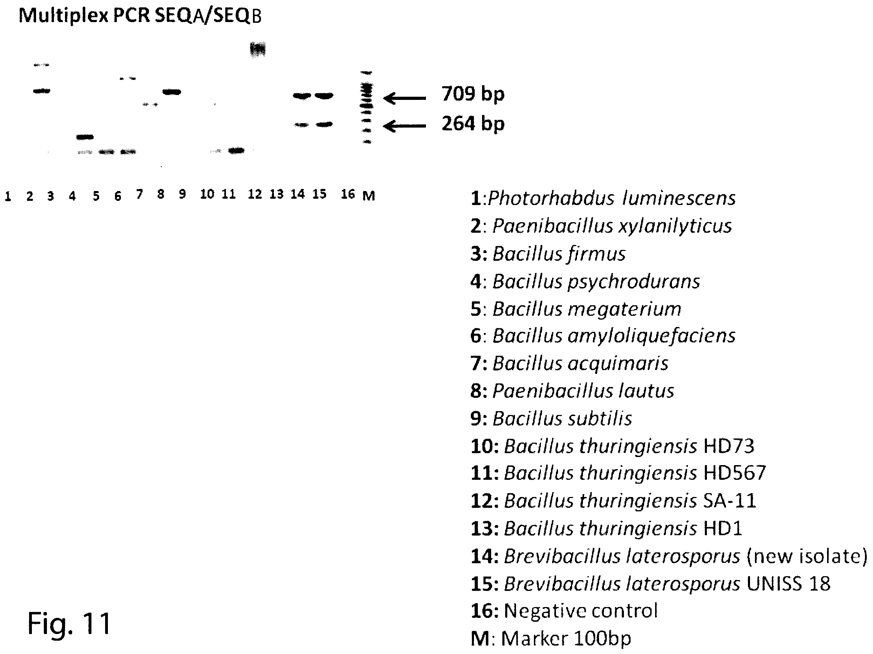

**Figure 11** shows the Multiplex PCR results to detect sequences A e B simultaneously on DNA extracted with commercial kit from different bacterial species: (1) *Photorhabdus luminescens,* (2) *Paenibacillus xylanilyticus,* (3) *Bacillus firmus,* (4) *Bacillus psychrodurans,* (5) *Bacillus megaterium,* (6) *Bacillus amyloliquefaciens,* (7) *Bacillus acquimaris,* (8) *Paenibacillus lautus,* (9) *Bacillus subtilis,* (10) *Bacillus thuringiensis* HD73, (11) *Bacillus thuringiensis* HD567, (12) *Bacillus thuringiensis* SA-11, (13) *Bacillus thuringiensis* HD1, (14) *Brevibacillus laterosporus* (new isolate), (15) *Brevibacillus laterosporus* UNISS 18 (16) Negative control; (M) Marker 100 bp. For sequence A the PCR products of 709 bp were obtained using C28s primers pair; for sequence B the PCR products of 264 bp were obtained using C14d primers pair.

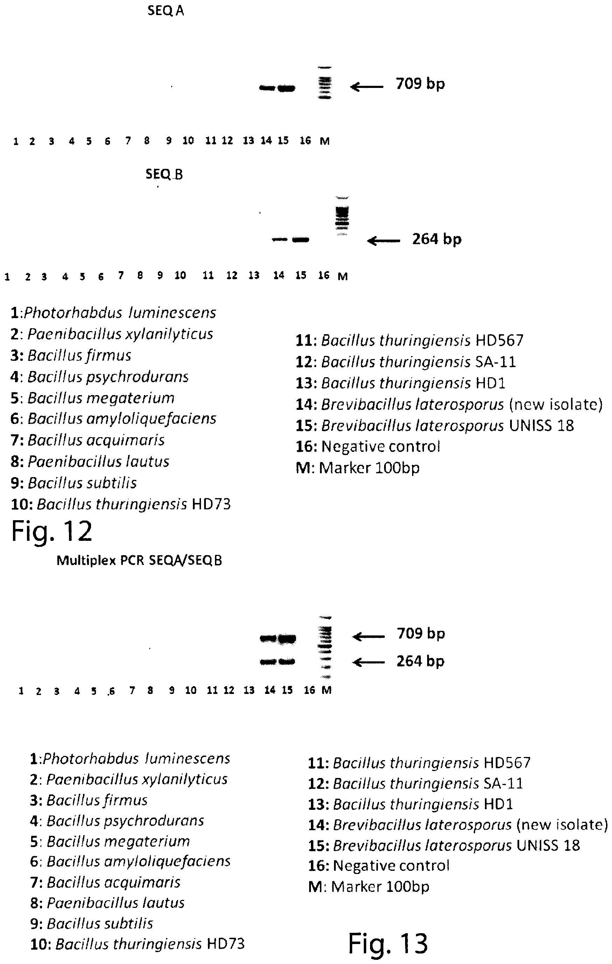

**Figure 12** shows the PCR results to detect sequences A and B on DNA extracted with boiling method from different bacterial species: (1) *Photorhabdus luminescens,* (2) *Paenibacillus xylanilyticus,* (3) *Bacillus firmus,* (4) *Bacillus psychrodurans,* (5) *Bacillus megaterium,* (6) *Bacillus amyloliquefaciens,* (7) *Bacillus acquimaris,* (8) *Paenibacillus lautus,* (9) *Bacillus subtilis,* (10) *Bacillus thuringiensis* HD73, (11) *Bacillus thuringiensis* HD567, (12) *Bacillus thuringiensis* SA-11, (13) *Bacillus thuringiensis* HD1, (14) *Brevibacillus laterosporus* (new isolate), (15) *Brevibacillus laterosporus* UNISS 18 (16) Negative control; (M) Marker 100 bp. For sequence A the PCR products of 709 bp were obtained using C28s primers pair; for sequence B the PCR products of 264 bp were obtained using C14d primers pair.
**Figure 13** shows the Multiplex PCR results to detect sequences A and B simultaneously on DNA extracted with boiling method from different bacterial species: (1) *Photorhabdus luminescens,* (2) *Paenibacillus xylanilyticus,* (3) *Bacillus firmus,* (4) *Bacillus psychrodurans,* (5) *Bacillus megaterium,* (6) *Bacillus amyloliquefaciens,* (7) *Bacillus acquimaris,* (8) *Paenibacillus lautus,* (9) *Bacillus subtilis,* (10) *Bacillus thuringiensis* HD73, (11) *Bacillus thuringiensis* HD567, (12) *Bacillus thuringiensis* SA-11, (13) *Bacillus thuringiensis* HD1, (14) *Brevibacillus laterosporus* (new isolate), (15) *Brevibacillus laterosporus* UNISS 18 (16) Negative control; (M) Marker 100 bp. For sequence A the PCR products of 709 bp were obtained using C28s primers pair; for sequence B the PCR products of 264 bp were obtained using C14d primers pair.

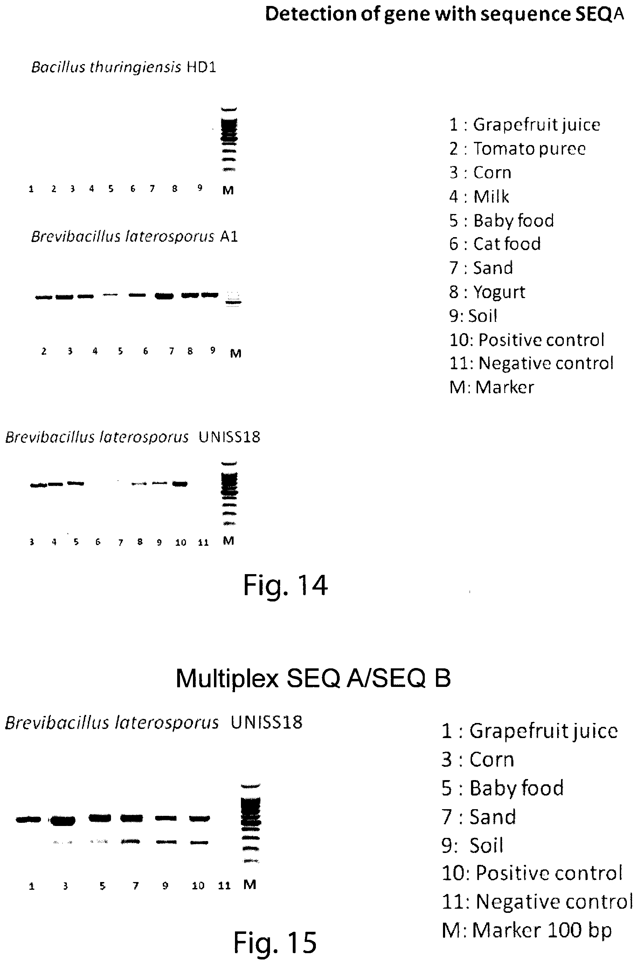

**Figure 14** shows the PCR results to detect sequence A on different matrices mixed with *Bacillus thuringiensis* HD1, *Brevibacillus laterosporus* A1 and *Brevibacillus laterosporus* UNISS 18. The matrices used were: (1) Grapefruit juice, (2) Tomato puree, (3) Corn, (4) Milk, (5) Baby food, (6) Cat food, (7) Sand, (8) Yougurt, (9) Soil, (10) Positive control, (11) Negative control; (M) Marker 100 bp. The sequence A PCR products of 709 bp were obtained using C28s primers pair.
**Figure 15** shows the Multiplex PCR results to detect SEQ ID NO: 30 and SEQ ID NO: 37 simultaneously on different matrices mixed with *Brevibacillus laterosporus* UNISS 18. The matrices used were: (1) Grapefruit juice, (3) Corn, (5) Baby food, (7) Sand, (9) Soil, (10) Positive control, (11) Negative control; (M) Marker 100 bp. For SEQ ID NO: 30 the PCR products of 709 bp were obtained using C28s primers pair; for SEQ ID NO:37 the PCR products of 264 bp were obtained using C14d primers pair.

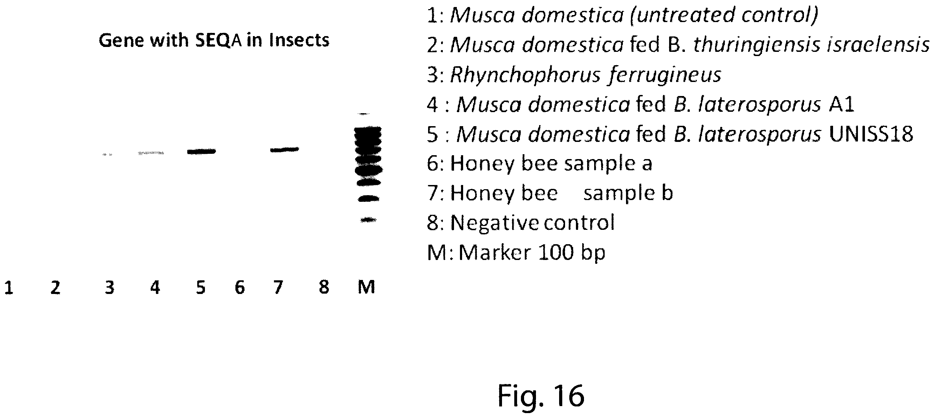

**Figure 16** shows the PCR results to detect sequence A on different insect matrices. The insects used were: (1) *Musca domestica* (untreated control), (2) *Musca domestica* fed B. *thuringiensis israelensis,* (3) *Rhynchophorus ferrugineus,* (4) *Musca domestica* fed *Brevibacillus laterosporus* A1, (5) *Musca domestica* fed *Brevibacillus laterosporus* UNISS18, (6) Honey bee sample a, (7) Honey bee sample b , (8) Negative control; (M) Marker 100 bp. Sequence A PCR products of 709 bp were obtained using C28s primers pair.

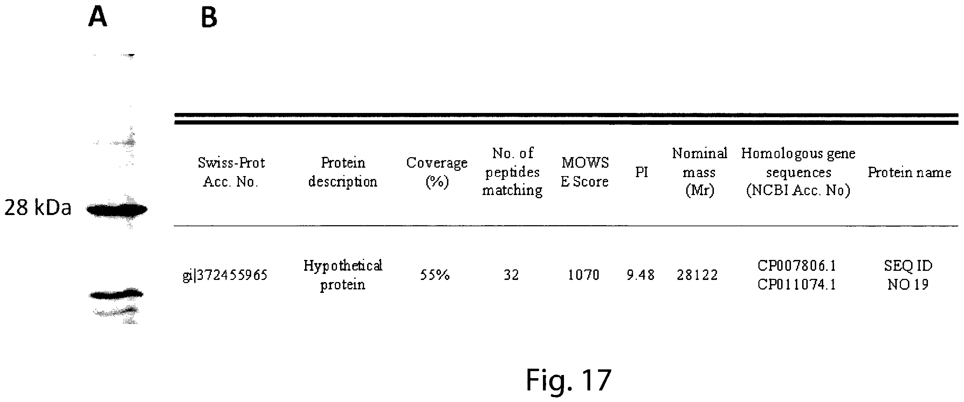

**Figure 17** shows 1-DE protein profile of *Brevibacillus laterosporus SC-CSPB* fraction extract showing a main band with a MW of around 28 kDa (A) and the results of its identification by LC-MS/MS (B).

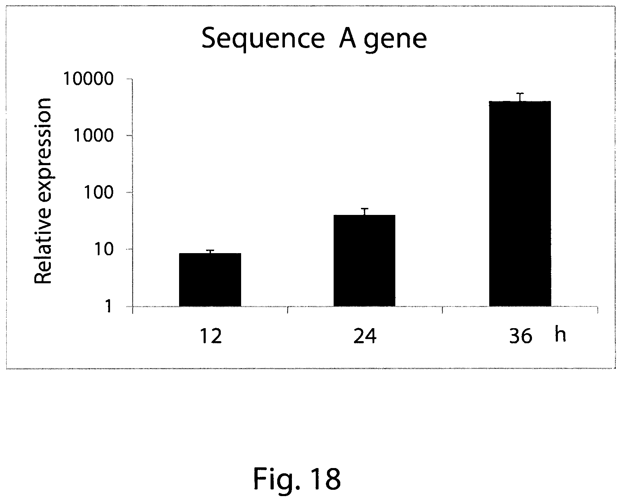

**Figure 18** shows the relative expression level of sequence A gene during different *Brevibacillus laterosporus* stages of growth: vegetative phase (12 h), stationary phase (24 h), sporulation phase (36 h). Relative over time changes are expressed as differences (delta) with the reference gene (16S rRNA), and are expressed as 2^(-deltaCt).

### Example 1: Identification of two Brevibacillus laterosporus genes for its species-specific detection

Proteins of the complex including the spore coat and the canoe shaped parasporal body (SC-CSPB-complex) of Brevibacilus laterosporus strain NCIMB 41419 (UNISS 18) have been extracted by alkali. The proteins have been extracted by bringing the pH of a clean spore suspension to 11.5 by the careful addition of 0.2 N NaOH. Then an equal volume of 2 % thioglycollic acid at the same pH was added and mixed. After 10 min incubation at room temperature, the suspension was centrifuged at 11,000g and 4°C for 15 min, and the supernatant was collected before being dialysed at 4°C against 50 mM Tris-CI pH 8.0, using SnakeSkin™ Pleated Dialysis tubing, 3,500 MWCO (Cole-Parmer Instrument Company, UK). The washing buffer was changed three times after 1 h, 2 h and overnight. All insoluble material was removed from the sample by centrifugation at 20,000g for 15 min. The final sample was analyzed by 1D-PAGE and major bands with a molecular weight ranging between 12 and 30 kDa were cut and subjected to *in situ* tryptic digestion before analyzes by LC MS/MS. Mass spectrometry output data were analyzed employing a Mascot server (Matrix Science, London, UK) and processed against the NCBI database (http://www.ncbi.nlm.nih.gov). In this way, among other proteins, two were identified as corresponding to hypothetical proteins whose localization in the bacterial cell and function was unknown. The previous knowledge of the sequences of the genes encoding for these two proteins is the result of the whole or partial genome sequencing of strains LMG 15441, GI-9, PE36, B9, and DSM 25.

In order to develop a system to detect *Brevibacillus laterosporus* among genes encoding for proteins associated to the SC-CSPB complex, two species-specific genes were identified. More specifically, one of the two sequences encodes for a protein of SC-CSPB complex with a molecular weight around 28kDa (sequence A), while the other sequence encodes for a protein of SC-CSPB complex with a molecular weight around 14 kDa (sequence B).

Both sequences of different Brevibacillus laterosporus strains are shown below:

### Sequence encoding for a protein of SC-CSPB complex with a molecular weight around 28kDa (sequence A)

### Brevibacillus laterosporus LMG 15441 (ATCC 9141), GenBank: CP007806.1

### ACCESSION CP007806 REGION: 373080..373850 /protein_id="AIG24770.1 "

Antisense sequence SEQ ID NO:1
Sense sequence SEQ ID NO:21

### Brevibacillus laterosporus GI-9, GenBank: GenBank: CAGD01000026.1

### ACCESSION CAGD01000026 REGION: 56770..57540 /protein_id="CCF16244.1 "

Antisense sequence SEQ ID NO:22
Sense sequence SEQ ID NO:23

### Brevibacillus laterosporus PE36, GenBank: AXBT01000039.1

### ACCESSION AXBT01000039 REGION: 14681..15451 /protein_id="ERM16574.1 "

Antisense sequence SEQ ID NO:24
Sense sequence SEQ ID NO:25

### Brevibacillus laterosporus strain B9, GenBank: JNFS01000001.1

### ACCESSION JNFS01000001 REGION: 1072206..1072976

Antisense sequence SEQ ID NO:26
Sense sequence SEQ ID NO:27

### Brevibacillus laterosporus DSM 25, GenBank: ARFS01 000034. 1

### ACCESSION ARFS01000034 REGION: 57186..57956

Antisense sequence SEQ ID NO:28
Sense sequence SEQ ID NO:29

### Brevibacillus laterosporus NCIMB 41419 (UNISS 18)

Antisense sequence
   (SEQ ID NO:30)
UNISS 18 Sense sequence
   SEQ ID NO:31

Figure 1 shows the alignment of the above-mentioned antisense sequences proving that the sequences have high identity among different *Brevibacillus laterosporus* strains. The alignment has been carried out by Basic Local Alignment-Search Tool (BLAST) of the National Center for Biotechnology Information (NCBI).

In addition, Figure 2 shows the alignment of the polypeptide sequences codified by the above mentioned nucleic sequences.

### Sequence encoding for a protein of SC-CSPB complex with a molecular weight around 14 kDa (sequence B)

### Brevibacillus laterosporus GI-9, GenBank: CAGD01000037.1

### ACCESSION CAGD01000037 REGION: 7630..8049 /protein_id="CCF16818.1 "

Antisense sequence SEQ ID NO:2
Sense sequence SEQ ID NO:32

### Brevibacillus laterosporus PE36, GenBank: AXBT01000062.1

### ACCESSION AXBT01000062 REGION: 312171..312590 /protein_id="ERM16028.1 "

Antisense sequence SEQ ID NO:33
Sense sequence SEQ ID NO:34

### Brevibacillus laterosporus strain B9, GenBank: JNFS01000003.1

### ACCESSION JNFS01000003 REGION: 927027..927446

Antisense sequence SEQ ID NO:35
Sense sequence SEQ ID NO:36

### Brevibacillus laterosporus NCIMB 41419 (UNISS 18)

Antisense sequence (SEQ ID NO:37)

### UNISS18

Sense sequence SEQ ID NO:38

Figure 3 shows the alignment of the above-mentioned antisense sequences proving that the sequences have high identity among different *Brevibacillus laterosporus* strains. The alignment has been carried out by Basic Local Alignment-Search Tool (BLAST) of the National Center for Biotechnology Information (NCBI).

In addition, Figure 4 shows the alignment of the polypeptide sequences codified by the above mentioned nucleic sequences.

### Example 2: Design of PCR primers on Brevibacillus laterosporus species-specific genes with sequences SEQ ID NO:1 and SEQ ID NO:2

In order to develop a system to detect *Brevibacillus laterosporus,* pairs of primers were designed to amplify regions of different size within the sequences SEQ ID NO:1 and SEQ ID NO:2

The sequences SEQ ID NO:1 and SEQ ID NO:2, used to design the primers, are shown below:

### SEQ ID NO: 1

### Brevibacillus laterosporus LMG 15441 (ATCC 9141), GenBank: CP007806.1

ACCESSION CP007806 REGION: 373080..373850

### SEQ ID NO: 2

### Brevibacillus laterosporus GI-9, GenBank: CAGD01000037.1

ACCESSION CAGD01000037 REGION: 7630..8049

The primers sequences used to amplify regions of the gene with sequence SEQ ID NO: 1 are shown below:
**C28q F** 5'-GCTTCACACGATCAGCAACC-3' (SEQ ID NO: 3)
**C28q R** 5'-TGTAGGCGGGCAGCTAAAAA -3' (SEQ ID NO: 4);
   PCR product size 155 bp
**C28d1 F** 5'-C AGC TTG GTT CAC TTT ATT CG-3' (SEQ ID NO: 5)
**C28d1 R** 5'-TG AAG CAA GCA GGT AGT GAA-3' (SEQ ID NO: 6)
   PCR product size: 225 bp
**C28d2 F** 5'-CGT TTT TAC TTC TTG TCT TCC TAG-3'(SEQ ID NO: 7)
**C28d2 R** 5'-AG GTT GCT GAT CGT GTG A-3' (SEQ ID NO: 8)
   PCR product size: 269 bp
**C28d3 F** 5'-AGC CTT AGC ACC TGT ATC TTT G-3'(SEQ ID NO: 9)
**C28d3 R** 5'-AG GCA GCT ACT GAA GCT GA-3'(SEQ ID NO: 10)
   PCR product size: 536 bp
**C28s F** 5'-CTGCTACTAGTTGATCTAAG-3' (SEQ ID NO: 11)
**C28s R** 5'-CTGATTGGTAGCTTAGGTA-3'(SEQ ID NO: 12);
   PCR product size: 709 bp

The primers sequences used to amplify regions of the gene with sequence SEQ ID NO:2 are shown below:
**C14d F** 5'-CTA TTT ACA CGG GCG TAC G-3'(SEQ ID NO: 13)
**C14d R** 5'-CAT TCT TTT GAA AGTAGTAAG AAG-3' (SEQ ID NO: 14); Product size 264 bp
**C14q 1F** 5'-TCACCAAGACACAAAGCCCT-3'(SEQ ID NO: 15)
**C14q 1R** 5'-CTCTTTGCCGTAGATTCGCG-3'(SEQ ID NO: 16);
   Product size 193 bp
**C14q 2F** 5'-TCAGGGCATCACGTACACTT-3' (SEQ ID NO: 17)
**C14q 2R** 5'-GGGCTTTGTGTCTTGGTGAG-3' (SEQ ID NO: 18)
   Product size 195 bp

In order to develop a detection system of *Brevibacillus laterosporus,* each primers pair was designed on the sequences SEQ ID NO:1 (ACCESSION CP007806 REGION: 373080..373850) and SEQ ID No:2 (ACCESSION CAGD01000037 REGION: 7630..8049) genes. Later these primers were tested in PCR reactions to amplify DNA extracted from a *Brevibacillus laterosporus* UNISS 18 overnight culture. PCR reactions were set in a total volume of 25 µl containing: 1X reaction buffer; 1.5 mM of MgCl2+; 100 ng DNA; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. In figure 5 the PCR results show the expected band for each primers pair and since unspecific amplifications were not found the primers sequences designed for SEQ ID NO:1 and SEQ ID NO:2 can be used to detect *Brevibacillus laterosporus.*

### Example 3: Validation of C28s and C14d primers efficiency by PCR amplification of the Brevibacillus laterosporus DNA extracted with commercial kit

In order to test the efficiency of C28s and C14d primers pairs by PCR amplification, genomic DNA was extracted using commercial kit from five different *Brevibacillus laterosporus* strains: *Brevibacillus laterosporus* ATCC9141 (A1), *Brevibacillus laterosporus* ATCC6456 (A5), *Brevibacillus laterosporus* ATCC55122 (BOD), *Brevibacillus laterosporus* NI *(new isolate), Brevibacillus laterosporus* NCIMB 41419 (UNISS18). C28s and C14d primers pairs were used to detect genes with the sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa, in two different PCR reactions. For each strain the two PCR reactions were set in a total volume of 25 µl containing: 1X reaction buffer; 1.5 mM of MgCl2+; 100 ng DNA; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. Figure 6 shows the PCR results on agarose gels: the expected band for the region of 709 bp of the sequence A encoding for a protein of SC-CSPB complex with a molecular weight around 28kDa (for instance SEQ ID NO:1 of *Brevibacillus laterosporus* LMG 15441 or the corresponding sequences of other *Brevibacillus laterosporus* strains) was detected in all *Brevibacillus laterosporus* strains, whereas band for the region of 264 bp of the sequence B encoding for a protein of SC-CSPB complex with a molecular weight around 14 kDa (for instance SEQ ID NO:2 of *Brevibacillus laterosporus* GI-9 or the corresponding sequences of other *Brevibacillus laterosporus* strains) was found in *Brevibacillus laterosporus* NI and *Brevibacillus laterosporus* UNISS18. Since unspecific amplification was not found, the results demonstrate the efficiency of both C28s and C14d primers in detecting genes with the above mentioned sequences.

### Example 4: Validation of C28s and C14d primers efficiency by multiplex PCR amplification of the Brevibacillus laterosporus DNA extracted with commercial kit.

The efficiency of C28s and C14d primers pairs was also tested by multiplex PCR reactions. Therefore genomic DNA was extracted using commercial kit from five different *Brevibacillus laterosporus* strains: *Brevibacillus laterosporus* ATCC9141 (A1), *Brevibacillus laterosporus* ATCC6456 (A5), *Brevibacillus laterosporus* ATCC55122 (BOD), *Brevibacillus laterosporus* NI *(new isolate), Brevibacillus laterosporus* NCIMB 41419 (UNISS18). Multiplex PCR reactions were set up for the simultaneous detection of both genes with the sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa, using C28s and C14d primers pairs. For each strain the PCR reaction was set in a total volume of 25 µl containing: 1X reaction buffer; 1.5 mM of MgCl2+; 100 ng DNA; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. Figure 7 shows multiplex PCR results on agarose gel: expected bands for genes with the sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa were both detected in *Brevibacillus laterosporus* NI and *Brevibacillus laterosporus* UNISS18. In the other strains only the band relative to the gene with the sequence A encoding for a protein of SC-CSPB complex with a molecular weight around 28kDa was detected. The absence of unspecific bands confirms the efficiency of C28s and C14d primers pairs.

### Example 5: Validation of C28s and C14d primers efficiency by PCR amplification of the Brevibacillus laterosporus DNA extracted with boiling method.

To confirm the efficiency of C28s and C14d primers pairs by PCR amplification, genomic DNA from 5 different *Brevibacillus laterosporus* strains (*Brevibacillus laterosporus* ATCC9141 (A1), *Brevibacillus laterosporus* ATCC6456 (A5), *Brevibacillus laterosporus* ATCC55122 (BOD), *Brevibacillus laterosporus* NI *(new isolate), Brevibacillus laterosporus* NCIMB41419 (UNISS18) was extracted with boiling method. The DNA extraction was set up as follows: 1mL of each overnight culture was boiled at 100°C for 10 min and centrifuged at 12000 rpm for 5 min. The supernatant obtained was used as PCR template and C28s and C14d primers pairs were used to detect genes with sequences A and B (for instance SEQ ID NO:1 and SEQ ID NO:2 of *Brevibacillus laterosporus* LMG 15441 and of *Brevibacillus laterosporus* GI-9, respectively,or the corresponding sequences of other *Brevibacillus laterosporus* strains) encoding for a protein of SC-CSPB complex with a molecular weight around 28kDa and for a protein of SC-CSPB complex with a molecular weight around 14 kDa, respectively, in two different PCR reactions. Therefore for each strain the two PCR reactions were set in a total volume of 25 µl containing: 1X reaction buffer; 1.5 mM of MgCl2+; 2 µl of DNA templete; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. Figure 8 shows the PCR results on agarose gels: expected band of 709 bp for gene with sequence A encoding for a protein of SC-CSPB complex with a molecular weight around 28kDa (for instance SEQ ID NO:1 of *Brevibacillus laterosporus* LMG 15441 or the corresponding sequences of other Brevibacillus laterosporus strains) was detected in all *Brevibacillus laterosporus* strains, whereas the band of 264 bp for gene with sequence B encoding for a protein of SC-CSPB complex with a molecular weight around 14 kDa (for instance SEQ ID NO:2 of *Brevibacillus laterosporus* GI-9 or the corresponding sequences of other *Brevibacillus laterosporus* strains) was found in *Brevibacillus laterosporus* NI and *Brevibacillus laterosporus* UNISS18. Since unspecific amplification was not found, the results demonstrate the efficiency of both C28s and C14d primers in detecting genes with the above mentioned sequences.

### Example 6: Validation of C28s and C14d primers efficiency by multiplex PCR amplification of the Brevibacillus laterosporus DNA extracted with boiling method.

The efficiency of C28s and C14d primers pairs was also tested by multiplex PCR reactions, using DNA extracted with boiling method. Therefore genomic DNA from 5 different *Brevibacillus laterosporus* strains (*Brevibacillus laterosporus* ATCC9141 (A1), *Brevibacillu laterosporus* ATCC6456 (A5), *Brevibacillus laterosporus* ATCC55122 (BOD), *Brevibacillus laterosporus* NI *(new isolate), Brevibacillus laterosporus* NCIMB 41419 (UNISS18) was extracted with boiling method as follows: 1mL of each overnight culture was boiled at 100°C for 10 min and centrifuged at 12000 rpm for 5 min. The supernatant obtained was used as PCR template. Therefore multiplex PCR reactions were set up for the simultaneous detection of both genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa, using C28s and C14d primers pairs. For each strain the PCR reaction was set in a total volume of 25 µl containing: 1X reaction buffer; 1.5 mM of MgCl2+; 2 µl of DNA templete; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57 °C for 45 s and 72 °C for 45 s, followed by a final extension at 72 °C for 10 min. Figure 9 shows multiplex PCR results on agarose gels: expected bands corresponding to genes with sequences A and B encoding for a protein of SC-CSPB complex with a molecular weight around 28kDa and for a protein of SC-CSPB complex with a molecular weight around 14 kDa were both detected in *Brevibacillus laterosporus* NI and *Brevibacillus laterosporus* UNISS18. In the other strains only the band corresponding to the gene with sequence A encoding for a protein of SC-CSPB complex with a molecular weight around 28kDa was detected. The absence of unspecific bands confirms the efficiency of C28s and C14d primers pairs.

### Example 7: Validation of the species-specificity of C28s and C14d primers by PCR amplification of bacterial DNA extracted with commercial kit.

In order to validate the species-specificity of C28s and C14d primers pairs by PCR amplification, genomic DNA was extracted, using commercial kit, from the following bacterial species : *Photorhabdus luminescens, Paenibacillus xylanilyticus, Bacillus firmus, Bacillus psychrodurans, Bacillus megaterium, Bacillus amyloliquefaciens, Bacillus acquimaris, Paenibacillus lautus, Bacillus subtilis, Bacillus thuringiensis* HD73, *Bacillus thuringiensis* HD567, *Bacillus thuringiensis* SA-11, *Bacillus thuringiensis* HD1, *Brevibacillus laterosporus* (new isolate), *Brevibacillus laterosporus* NCIMB41419 (UNISS 18). C28s and C14d primers pairs were used to detect genes with sequences SEQ ID NO:1 and SEQ ID NO:2 respectively, in two different PCR reactions. For each species the two PCR reactions were set in a total volume of 25 µl containing: 1X reaction buffer; 1.5 mM of MgCl2+; 100 ng DNA; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. In Figure 10 the PCR results show that the bands of the expected size corresponding to genes with sequences A and B encoding for a protein of SC-CSPB complex with a molecular weight around 28kDa and for a protein of SC-CSPB complex with a molecular weight around 14 kDa were obtained only for *Brevibacillus laterosporus* strains. Therefore the species-specificity of these primers for *Brevibacillus laterosporus* DNA detection was demonstrated.

### Example 8: Validation of the species-specificity of C28s and C14d primers by multiplex PCR amplification of different bacterial DNA extracted with commercial kit.

The species- specificity of C28s e C14d primers pairs was also tested by multiplex PCR reactions. Therefore genomic DNA was extracted, using commercial kit, from the following bacterial species: *Photorhabdus luminescens, Paenibacillus xylanilyticus, Bacillus firmus, Bacillus psychrodurans, Bacillus megaterium, Bacillus amyloliquefaciens, Bacillus acquimaris, Paenibacillus lautus, Bacillus subtilis, Bacillus thuringiensis* HD73, *Bacillus thuringiensis* HD567, *Bacillus thuringiensis* SA-11, *Bacillus thuringiensis* HD1, *Brevibacillus laterosporus* (new isolate), *Brevibacillus laterosporus* NCIMB41419 (UNISS 18). Multiplex PCR reactions were set up for the simultaneous detection of both genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa , using C28s and C14d primers pairs. For each microorganism the PCR reaction was set in a total volume of 25 µl containing: 1X reaction buffer; 1.5 mM of MgCl2+; 100 ng DNA; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. In Figure 11 the multiplex PCR results show the expected bands corresponding to genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa that were detected in *Brevibacillus laterosporus* strains. Therefore the species-specificity of these primers for *Brevibacillus laterosporus* DNA detection was confirmed.

### Example 9: Validation of the species-specificity of C28s and C14d primers by PCR amplification of different bacterial DNA extracted with boiling method.

The species-specificity of C28s and C14d primers pairs was also tested by PCR amplification of genomic DNA extracted with boiling method from different bacterial species. Therefore the genomic DNA was extracted from the following bacterial species : *Photorhabdus luminescens, Paenibacillus xylanilyticus, Bacillus firmus, Bacillus psychrodurans, Bacillus megaterium, Bacillus amyloliquefaciens, Bacillus acquimaris, Paenibacillus lautus, Bacillus subtilis, Bacillus thuringiensis* HD73, *Bacillus thuringiensis* HD567, *Bacillus thuringiensis* SA-11, *Bacillus thuringiensis* HD1, *Brevibacillus laterosporus* (new isolate), *Brevibacillus laterosporus NCIMB 41419 (*UNISS 18). For each species the DNA extraction was set up as follows: 1mL of each overnight culture was boiled at 100°C for 10 min and centrifuged at 12000 rpm for 5 min. The supernatant obtained was used as PCR template and C28s and C14d primers pairs were used to detect genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other Brevibacillus laterosporus strains) encoding for a protein of SC-CSPB complex with a molecular weight around 28kDa and for a protein of SC-CSPB complex with a molecular weight around 14 kDa, respectively, in two different PCR reactions. Therefore for each species the two PCR reactions were set in a total volume of 25 µl containing: 1X reaction buffer; 1.5 mM of MgCl2+; 2 µl of DNA template; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57 °C for 45 s and 72 °C for 45 s, followed by a final extension at 72 °C for 10 min. In Figure 12 the PCR results show the bands of the expected size corresponding to genes with sequences A and B encoding for a protein of SC-CSPB complex with a molecular weight around 28kDa and for a protein of SC-CSPB complex with a molecular weight around 14 kDa only for *Brevibacillus laterosporus* strains. Therefore the species-specificity of these primers for *Brevibacillus laterosporus* DNA detection was confirmed.

### Example 10: Validation of the species-specificity of C28s and C14d primers by multiplex PCR amplification of different bacterial DNA extracted with boiling method.

The species-specificity of C28s and C14d primers pairs was also tested by multiplex PCR amplification of genomic DNA extracted with boiling method from different bacterial species. Therefore the genomic DNA was extracted from the following bacterial species : *Photorhabdus luminescens, Paenibacillus xylanilyticus, Bacillus firmus, Bacillus psychrodurans, Bacillus megaterium, Bacillus amyloliquefaciens, Bacillus acquimaris, Paenibacillus lautus, Bacillus subtilis, Bacillus thuringiensis* HD73, *Bacillus thuringiensis* HD567, *Bacillus thuringiensis* SA-11, *Bacillus thuringiensis* HD1, *Brevibacillus laterosporus* (new isolate), *Brevibacillus laterosporus NCIMB 41419* (UNISS 18). For each species the DNA extraction was set up as follows: 1mL of each overnight culture was boiled at 100°C for 10 min and centrifuged at 12000 rpm for 5 min. The supernatant obtained was used as PCR template. Therefore a multiplex PCR reactions were set up for the simultaneous detection of both genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other Brevibacillus laterosporus strains) encoding for a protein of SC-CSPB complex with a molecular weight around 28kDa and for a protein of SC-CSPB complex with a molecular weight around 14 kDa using C28s and C14d primers pairs. For each strain the PCR reaction was set in a total volume of 25 µl containing: 1X reaction buffer; 1.5 mM of MgCl2+; 2 µl of DNA templete; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. In Figure 13 the multiplex PCR results show the bands of the expected size corresponding to genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other Brevibacillus laterosporus strains) encoding for a protein of SC-CSPB complex with a molecular weight around 28kDa and for a protein of SC-CSPB complex with a molecular weight around 14 kDa in *Brevibacillus laterosporus* strains. Therefore the species-specificity of these primers for *Brevibacillus laterosporus* DNA detection was confirmed.

### Example 11: Validation of C28s and C14d primers for the detection of Brevibacillus laterosporus in different matrices

In order to validate C28s and C14d primers pairs for the detection of *Brevibacillus laterosporus,* matrices of different nature were chosen and mixed with *Brevibacillus laterosporus* cultures. The experiments were set up using the following matrices: grapefruit juice, tomato puree, corn, milk, baby food, cat food, yogurt, sand and soil; the bacterial culture used were: *Brevibacillus laterosporus* ATCC9141 (A1), *Brevibacillus laterosporus NCIMB 41419* (UNISS 18), *Bacillus thuringiensis* HD1 (negative control). For each experiment 1mL of liquid matrix or 1 gr of solid matrix was mixed with each bacterial culture listed above and containing ∼ 10*6 cell/mL. After mixing by vortex, the solution was used to extract genomic DNA with the boiling method as follows: 1mL of each solution was boiled at 100°C for 10 min and centrifuged at 12000 rpm for 5 min. The supernatant obtained was used as PCR template either in singleplex and multiplex PCR reaction with C28s and C14d pairs primers. Therefore the PCR reactions were set in a total volume of 25 µl containing: 1X reaction buffer; 1.5 mM of MgCl2+; 2 µl of DNA templete; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57°C for 45 s and 72 °C for 45 s, followed by a final extension at 72 °C for 10 min. In Figure 14 the PCR results show the detection of the expected band corresponding to the gene with sequence A encoding for a protein of SC-CSPB complex with a molecular weight around 28kDa in *Brevibacillus laterosporus* ATCC9141 (A1) and *Brevibacillus laterosporus* UNISS18 samples.

Figure 15 shows the detection genes with sequences A and B (for instance SEQ ID NO:1 and SEQ ID NO:2 of *Brevibacillus laterosporus* LMG 15441 and of *Brevibacillus laterosporus* GI-9, respectively, or the corresponding sequences of other *Brevibacillus laterosporus* strains) encoding for a protein of SC-CSPB complex with a molecular weight around 28kDa and for a protein of SC-CSPB complex with a molecular weight around 14 kDa in multiplex reaction. These results confirm that, among matrices from different nature, C28s and C14d primers pairs are efficient in detecting *Brevibacillus laterosporus.*

### Example 12: Validation of C28s primers for the detection of Brevibacillus laterosporus in insect

The efficiency of C28s and C14d primers pairs was tested in detecting *Brevibacillus laterosporus* in insects either *wild*-caught or *laboratory-*raised. Samples of *Apis mellifera* and *Rhynchophorus ferrugineus* were caught from the wild, whereas samples of *Musca domestica* were raised in laboratory on a diet lacking *Brevibacillus laterosporus* (untreated control) or on a diet containing either *Brevibacillus laterosporus* ATCC9141 (A1) or *Brevibacillus laterosporus NCIMB 41419* (UNISS18) or *Bacillus thuringiensis israelensis* (negative control). Each insect was ground in 1mL / 5mL of sterile MIIQ H2O and the supernatant was recovered by centrifugation. Later the DNA was extracted with the boiling method as follows: the recovered supernatant was boiled at 100°C for 10min, centrifuged at 12000 rpm for 5 min and 5 µl were used as PCR template. The PCR reaction was set up using the C28s primers pair in a total volume of 25 µl containing: 1X reaction buffer; 1.5 mM of MgCl2+; 0.4 µM of each primer; 0.2 µM of each dNTPs; and 0.75 U of Taq DNA Polymerase. The reaction conditions were as follows: initial denaturation at 95 °C for 5 min, followed by 35 cycles of 95 °C for 30 s, 57 °C for 45 s and 72 °C for 45 s, followed by a final extension at 72 °C for 10 min. In Figure 16 the PCR results show the band of 709 bp corresponding to the gene with sequence A (for instance SEQ ID NO:1 of *Brevibacillus laterosporus* LMG 15441 or the corresponding sequences of other *Brevibacillus laterosporus* strains) encoding for a protein of SC-CSPB complex with a molecular weight around 28kDa in samples of *Apis mellifera* and *Rhynchophorus ferrugineus.* In fact it is known that *Brevibacillus laterosporus* is a common inhabitant of the body of certain insects like *Apis mellifera.* In the case of *Musca domestica* the band of 709 bp corresponding to the gene with sequence A encoding for a protein of SC-CSPB complex with a molecular weight around 28kDa was obtained only for insect specimens fed with *Brevibacillus laterosporus.* The results confirm the efficiency of C28s primers pair in detecting *Brevibacillus laterosporus* in the insect body.

### Example 13: Validation of C28q primers efficiency by quantitative Real-Time PCR for the detection of Brevibacillus laterosporus in insects.

C28q primers were designed to detect *Brevibacillus laterosporus* by quantitative real-time PCR (qPCR) on DNA extracted from *Apis mellifera* adults. Therefore the total honey bee DNA was extracted with commercial kit, quantified and used to prepare DNA standards for qPCR. The efficiency of C28q primers was validated by six 2-fold serial dilution standards, starting from 100 ng/µl down to 3.125 ng/µl of total honey bee DNA. For each standard two different reactions were set up using C28q primers and EF1 primers separately, in order to detect specifically the *Brevibacillus laterosporus* gene with sequence A (for instance SEQ ID NO:1 of Brevibacillus laterosporus LMG 15441 or the corresponding sequences of other Brevibacillus laterosporus strains) encoding for a protein of SC-CSPB complex with a molecular weight around 28kDa and honey bee EF1 housekeeping gene. Then the qPCR reactions were performed in a total volume of 20 µl containing: 1X SYBR Green PCR Master Mix; 0.5 µM of each primer and 3 µl of DNA templete. For each standard point the Ct values were detected for the *Brevibacillus laterosporus* gene with sequence A encoding for a protein of SC-CSPB complex with a molecular weight around 28kDa and *Apis mellifera EF1* housekeeping gene as show in Table 1. These results demonstrate that C28q primers can be used in quantitative Real-Time PCR methods to detect *Brevibacillus laterosporus* DNA from a pool of total insect DNA.

**Table 1**

| **Gene Detected** | **Task** | **Quantity** | **Ct value** | **DNA starting concentration** | **DNA final concentration** |
|---|---|---|---|---|---|
| Brevibacillus laterosporus SEQ N.1 | Negative control | **0** | **Undetermined** | | |
| Brevibacillus laterosporus SEQ N.1 | Standard | **1** | **26.93717** | 100 ng/ µl | 15 ng/ µl |
| Brevibacillus laterosporus SEQ N.1 | Standard | **0.5** | **27.585375** | 50 ng/ µl | 7,5 ng/ µl |
| Brevibacillus laterosporus SEQ N.1 | Standard | **0.25** | **28.142847** | 25 ng/ µl | 3,75 ng/ µl |
| Brevibacillus laterosporus SEQ N.1 | Standard | **0.125** | **29.046078** | 12,5 ng/ µl | 1,875 ng/ µl |
| Brevibacillus laterosporus SEQ N.1 | Standard | **0.062** | **30.138021** | 6,25 ng/ µl | 0, 937ng/ µl |
| Brevibacillus laterosporus SEQ N.1 | Standard | **0.031** | **30.383854** | 3,125 ng/ µl | 0,468 ng/ µl |
| | | | | | |
| Apis mellifera E1F | Negative control | **0** | **Undetermined** | | |
| Apis mellifera E1F | Standard | **1** | **21.931072** | 100 ng/ µl | 15 ng/ µl |
| Apis mellifera E1F | Standard | **0.5** | **22.682041** | 50 ng/ µl | 7,5 ng/ µl |
| Apis mellifera E1F | Standard | **0.25** | **23.612026** | 25 ng/ µl | 3,75 ng/ µl |
| Apis mellifera E1F | Standard | **0.125** | **24.441343** | 12,5 ng/ µl | 1, 875 ng/ µl |
| Apis mellifera E1F | Standard | **0.062** | **25.427956** | 6,25 ng/ µl | 0, 937ng/ µl |
| Apis mellifera E1F | Standard | **0.031** | **26.397243** | 3,125 ng/ µl | 0,468 ng/ µl |
| Brevibacillus laterosporus SEQ N.1 | Negative control | **0** | **Undetermined** | | |

### Example 14: Identification of spore surface proteins corresponding to SEQ ID NO 19 on Brevibacillus laterosporus spores

Synchronized cultures of Brevibacillus laterosporus strain UNISS 18 were used for surface proteins extraction and separation from spores. Proteins from the spore coat-canoe shaped parasporal body complex (SC-CSPB) were extracted from pure spore suspensions harvested by centrifugation at 13,000 rpm for 10 min, and re-suspended in lml 0.1N NaOH - 1% thioglycollic acid. The suspension was titrated adding 1 M NaOH until pH 11.5 and centrifuged at 13,000 rpm for 10 min. The supernatant was dialysed at 4°C against water using SnakeSkin™ Pleated Dialysis tubing, 3,500 MWCO (Cole-Parmer Instrument Company, UK). Water was changed three times after 1 h, 2 h and overnight, and then the supernatant was collected for analysis. Protein samples were mixed with Laemmli buffer, boiled for 5 min and run in a 10% or 15 % SDS-PAGE gel using a Mini-Protrean electrophoresis system (BioRad Laboratories Inc., USA). Gels were stained with Coomassie and digitized with an ImageScanner III (GE Healthcare).

Individual gel regions corresponding to major protein bands from different polyacrylamide gels were manually excised, destained, reduced, carbamidomethylated, and trypsin digested. Tryptic peptides were submitted to LC MS/MS analysis using a XCT Ultra 6340 ion trap equipped with a 1200 HPLC system and a chip cube (Agilent Technologies, Palo Alto, CA). Mass spectrometry output data were analyzed on the software provided by the manufacturer (6300 Series Ion Trap LCMS) employing Mascot Daemon MS/MS ion search software (Version 2.3, Matrix Science, Boston, MA) for protein identification. Data were then processed against the NCBI database (http://www.ncbi.nlm.nih.gov).

As shown in Figure 17, the 1-DE profile of surface proteins specifically extracted by alkali and a reducing agent showed a major band corresponding to a molecular weight around 28 kDa. As a result of LC-MS/MS analysis, protein corresponding to SEQ ID NO 19 was identified.

This experiment demonstrates the actual and abundant expression of protein corresponding to SEQ ID NO 19, which confirms the possibility to target this protein through methods known in the art (for example ELISA, Western Blot) for the detection of Brevibacillus laterosporus, and especially Brevibacillus laterosporus spores.

### Example 15: Determination of the level of expression of gene with sequence A in comparison to 16S rRNA gene

The purpose of this experiment was to demonstrate the higher level of expression of gene corresponding to sequence A in respect to 16S rRNA gene in *Brevibacillus laterosporus,* in order to prove the achievement of a significantly higher efficiency of the method of the present invention targeting sequence A gene, in comparison to other detection methods based on 16S rRNA gene detection. The relative expression levels of sequence A gene and 16S rRNA gene mRNAs were examined by quantitative real-time PCR (qRT-PCR). For this purpose, total RNA was extracted from 24 h cultures of *Brevibacillus laterosporus* NCIMB41419 (UNISS18). The bacterial samples were immediately resuspended in TRlzol®Reagent (Life Technologies) before being subjected to sonication and cooling in ice. Then, extrated RNA (1 µg), treated with RQ1 RNase-Free DNase (Promega), was reverse transcribed to complementary DNA (cDNA) with SuperScript® II Reverse Transcriptase and RNaseOUT™ Recombinant Ribonuclease Inhibitor using a mix of oligo(dT) and random hexamer primers according to manufacturer's instructions (Life Technologies). Quantitative PCR experiments were carried out soon after the synthesis of cDNA. Reactions were conducted using *Power* SYBR® Green PCR Master Mix and were run on an Applied Biosystems 7900HT Fast Real-Time PCR System according to manufacturer's instructions (Life Technologies) and with following cycle conditions: 50°C 2 min, 95°C 10 min, 95 °C 15 s and 60 °C 1 min (40 cycles), 60°C 1 min. Used primers, respectively forward and reverse, were 5'-GCTTCACACGATCAGCAACC-3' (SEQ ID NO:3) and 5'-TGTAGGCGGGCAGCTAAAAA -3' (SEQ ID NO:4) for cDNA corresponding to sequence A, and 5'-TGTAGCGGTGAAATGCGTAG-3'(SEQ ID NO:47) and 5'-GCGGCACTAAGGGTATTGAA-3' (SEQ ID NO:48) designed on *Brevibacillus laterosporus* 16S rRNA gene (GeneBank Acc. NO. NR_112212) used as reference gene. The primers pairs efficiency was evaluated by standard curve and dissociation curve analyses, and according to the manufacturer's manual. For each primers pair the dissociation curve was set increasing gradually the temperature from 60°C to 95° C after the real-time PCR reaction. To exclude genomic DNA contaminations, reactions included controls lacking template or reverse transcriptase. Samples were run in three technical replicates. Three independent experiments with different batches of bacterial cultures were conducted. Real time qPCR data were analyzed using 1 Way-ANOVA followed by Least Significant Difference (LSD) tests for post-hoc comparison of means. As a result, sequence A gene showed a significantly higher level of expression in comparison to 16S rRNA (p < 0. 05). In terms of threshold cycle (Ct), means ± SE were as follow: 17.52 ± 0.44 for SEQ. A gene; 27.68 ± 0.71 for 16S rRNA.

It can be concluded that, since Brevibacillus laterosporus produces a higher number of mRNA copy (= higher expression) of SEQUENCE A gene in comparison to 16S rRNA gene, a higher efficiency in detecting or quantifying Brevibacillus laterosporus by RT-PCR or RT-qPCR, respectively, is achievable with the method of the present invention based on SEQUENCE A, in respect to methods known in the art and based on 16S rRNA.

### Example 16: Determination of the level of expression of genes with sequences A, during different bacterial stages

The purpose of this experiment was to determine the level of expression of gene corresponding to sequence A in *Brevibacillus laterosporus* during different bacterial stage of growth. For this purpose aliquots of synchronized bacterial cultures were harvested at consecutive time intervals (12, 24, 36 h), corresponding to exponential, stationary and sporulation phases, respectively, as confirmed by phase microscopy observations. The relative expression levels of sequence A gene and of the reference gene 16S rRNA were then examined by quantitative real-time PCR (qRT-PCR). For this purpose, total RNA was extracted from harvested cultures of *Brevibacillus laterosporus* NCIMB41419 (UNISS18). The bacterial samples were immediately resuspended in TRlzol®Reagent (Life Technologies) before being subjected to sonication and cooling in ice. Then, extrated RNA (1 µg), treated with RQ1 RNase-Free DNase (Promega), was reverse transcribed to complementary DNA (cDNA) with SuperScript® II Reverse Transcriptase and RNaseOUT™ Recombinant Ribonuclease Inhibitor using a mix of oligo(dT) and random hexamer primers according to manufacturer's instructions (Life Technologies). Quantitative PCR experiments were carried out soon after the synthesis of cDNA. Reactions were conducted using *Power* SYBR® Green PCR Master Mix and were run on an Applied Biosystems 7900HT Fast Real-Time PCR System according to manufacturer's instructions (Life Technologies) and with following cycle conditions: 50°C 2 min, 95°C 10 min, 95 °C 15 s and 60 °C 1 min (40 cycles), 60°C 1 min. Used primers, respectively forward and reverse, were 5'-GCTTCACACGATCAGCAACC-3' (SEQ ID NO:3) and 5'-TGTAGGCGGGCAGCTAAAAA -3' (SEQ ID NO:4) for cDNA corresponding to sequence A, and 5'-TGTAGCGGTGAAATGCGTAG-3' (SEQ ID NO: 47) and 5'-GCGGCACTAAGGGTATTGAA-3' (SEQ ID NO:48) designed on *Brevibacillus laterosporus* 16S rRNA gene (GeneBank Acc. NO. NR_112212) used as reference gene. The primers pairs efficiency was evaluated by standard curve and dissociation curve analyses, and according to the manufacturer's manual. For each primers pair the dissociation curve was set increasing gradually the temperature from 60°C to 95° C after the real-time PCR reaction. To exclude genomic DNA contaminations, reactions included controls lacking template or reverse transcriptase. Samples were run in three technical replicates. Three independent experiments with different batches of bacterial cultures were conducted. Real time qPCR data were analyzed using 1 Way-ANOVA followed by Least Significant Difference (LSD) tests for post-hoc comparison of means. As a result, SEQUENCE A gene showed a significant increase in the level of expression during the sporulation phase (p < 0. 05), as shown by Figure 18. It can be concluded that, the expression level of SEQUENCE A is always significantly higher than 16S rRNA and increases over bacterial growth toward sporulation, which is in relation to the increased synthesis of the encoded protein (SEQ ID NO 19)that is part of the spore coat-canoe shaped parasporal body complex. This means that the method of detection using primers designed on SEQUENCE A allows the detection of Brevibacillus laterosporus in any stage of growth and may also allow to monitor the state of bacterial growth.

### Example 17: Comparative experiment of Brevibacillus laterosporus detection using Sequence A and 16S rRNA based methods.

The purpose of this experiment was to show the higher efficiency of targeting Sequence A gene for the detection of *Brevibacillus laterosporus* in a matrix, in respect to targeting 16S rRNA. For this purpose, PCR amplification was based on cDNA obtained by RNA in vitro transcription. Total RNA was extracted from homogenized pools of 10 *Apis mellifera* workers employing TRlzol® Reagent (Life Technologies) according to manufacturer's protocol. All RNA samples were treated with RQ1 RNase-Free DNase (Promega) and an aliquot (1 µg) of each was used to synthesize first-strand cDNA employing oligo dT (Promega), SuperScript® II Reverse Transcriptase (Life Technologies) and RNaseOUT™ Recombinant Ribonuclease Inhibitor (Life Technologies) according to the manufacturers' instructions. Serial dilutions of the template (1:10; 1:100; 1:1000) were prepared by diluting cDNA samples with sterile water. The PCR reactions were set up in a total volume of 25 µl containing: 1X reaction buffer; 1.5 mM of MgCl2+; 0.4 µM of each primer; 0.2 µM of each dNTPs; and 0.75 U of Taq DNA Polymerase. The reaction conditions were as follows: initial denaturation at 95 °C for 5 min, followed by 30 cycles of 95 °C for 40 s, 61 °C for 40 s and 72 °C for 40 s, followed by a final extension at 72 °C for 10 min. An aliquot (1 µl) o the previously mentioned serial dilutions was used in different PCR reactions. The following primers pairs were used in different PCR reactions using the same templates: primers pair SEQ ID NO:11 and SEQ ID NO:12 targeting Sequence A ; primers pair SEQ ID NO:3 and SEQ ID NO:4 targeting Sequence A; primer set L25 5'-TGAAGCGAAACGGAAAG-3' (SEQ ID NO: 49) and R322 5'-CGTCAAGGTGCTACCTTATT-3' (SEQ ID NO: 50) targeting 16s rRNA (CN101956018A); primer pairs 5'-TGTAGCGGTGAAATGCGTAG-3' (SEQ ID NO:47) and 5'-GCGGCACTAAGGGTATTGAA-3' (SEQ ID NO:48) which have been designed on *Brevibacillus laterosporus* 16S rRNA gene (GeneBank Acc. NO. NR_112212); primer pairs BREV174F 5'-AGACCGGGATAACATAGGGAAACTTAT-3' (SEQ ID No:51) and 1377R 5'-GGCATGCTGATCCGCGATTACTAGC-3' (SEQ ID NO:52) targeting 16s rRNA (Shida et al., 1996).

Table 2 shows the higher detection efficiency when targeting SEQUENCE A instead of 16S rRNA, which is in relation to the higher expression level of Sequence A gene. Namely, table 2 shows the detection of *Brevibacillus laterosporus* from an insect based matrix (*Apis mellifera* workers) by reverse transcription polymerase chain reaction (RT-PCR) using different primer pairs targeting Sequence A or 16S rRNA and different dilutions of cDNA template.

**Table 2**

| PRIMER PAIRS | TARGET GENE | Reference | DETECTION RESULT | | |
|---|---|---|---|---|---|
| | | | (+) = positive | | |
| | | | (-) = negative | | |
| | | | Serial dilutions | | |
| | | | 1:10 | 1:100 | 1:1000 |
| 5'-CTGCTACTAGTTGATCTAAG-3' | *Sequence A* | SEQ ID NO:11 | + | + | + |
| and | | and | | | |
| 5'-CTGATTGGTAGCTTAGGTA-3' | | SEQ ID NO:12 | | | |
| | *Sequence A* | SEQ ID NO:3 | + | + | + |
| and | | and | | | |
| | | SEQ ID NO:4 | | | |
| 5'-TGAAGCGAAACGGAAAG-3' | *16S rRNA* | SEQ ID 49 (L25) and SEQ IDNO:50 (R322) | + | - | - |
| and | | | | | |
| 5'-CGTCAAGGTGCTACCTTATT-3' | | Patent (China) CN101956018A | | | |
| | *16S rRNA* | SEQ ID NO: 47 and 48 Designed on sequence with GeneBank Acc. NO. NR_112212 | + | - | - |
| and | | | | | |
| | | | | | |
| | *16S rRNA* | SEQ ID NO:51 (BREV174F) | + | - | - |
| and | | and | | | |
| | | SEQ ID NO: 52 (1377R) Shida et al., 1996 | | | |

The results of this experiment confirm the higher efficiency of the method of the present invention targeting Sequence A in detecting *Brevibacillus laterosporus,* in comparison with other detection methods based on 16S rRNA. In other terms, the method of the present invention allows to detect/quantify Brevibacillus laterosporus contained in a matrix at significantly lower concentrations.

### References

Djukic M., Poehlein A., Thürmer A., Daniel R., 2011. Genome sequence of Brevibacillus laterosporus LMG 15441, a pathogen of invertebrates. Journal of Bacteriology 193: 5535-5536.

Fits-James, P.C., Young, I.E., 1958. Morphological and chemical studies of the spores and parasporal bodies of Bacillus laterosporus. J. Biophys. Biochem. Cytol. 4, 639-649.

Li,G., Xu,J., Song,W., Ye,W., Dong,G., Zhu,L., Guo,L. Full genome sequence of Brevibacillus laterosporus strain B9; a biological control strain isolated from Zhejiang, China [Unpublished]. Submitted to gene bank (28-MAY-2014) State Key Lab for Rice Biology, China National Rice Research Institute, Tiyuchang Road 359, Hangzhou, Zhejiang 310006, China.

Ruiu L., Floris I., Satta A., Ellar D.J., 2007. Toxicity of a Brevibacillus laterosporus strain lacking parasporal crystals against Musca domestica and Aedes aegypti. Biological Control, vol. 43; p. 136-143.

Ruiu L., 2013. Brevibacillus laterosporus, a Pathogen of Invertebrates and a Broad-Spectrum Antimicrobial Species. Insects, vol. 4; p. 476-492.

Shida, O., Takagi, H., Kadowaki, K., Komagata K., 1996. Proposal for two new genera, Brevibacillus gen. nov. and Aneurinobacillus gen. nov. International Journal of Systematic Bacteriology 46: 939-946.

Sharma, V., Singh, P.K., Midha, S., Ranjan, M., Korpole, S., Patil, P.B., 2012. Genome sequence of Brevibacillus laterosporus strain GI-9 Journal of Bacteriology

Theodore, C.M., Stamps, B.W., King, J.B., Price, L.S.L., Powell, D.R., Stevenson, B.S., Cichewicz, R.H., 2014. Genomic and metabolomic insights into the natural product biosynthetic diversity of a feral-hog-associated Brevibacillus laterosporus strain. PLoS ONE 9 (3), e90124

## Claims

1. Method for the detection and/or quantification of *Brevibacillus laterosporus* in a sample, said method comprising or consisting of the detection and/or quantification of at least one marker selected from the group consisting of:
a nucleic acid sequence comprising or consisting of SEQ ID NO:1 or the complement nucleic acid sequence thereof; a fragment of said SEQ ID NO:1 or complement nucleic acid sequence thereof, said fragment having at least 12 bp, preferably from 15 to 30 bp, more preferably from 18 to 24 bp; a nucleic acid sequence having a sequence identity of at least 80%, preferably 90% with said SEQ ID NO: 1, complement nucleic acid sequence or said fragment;
a surface polypeptide sequence comprising or consisting of SEQ ID NO: 19, a fragment thereof having at least 5 aminoacids, preferably from 6 to 20 aminoacids, more preferably from 8 to 15 aminoacids;
a polypeptide sequence having a sequence identity of at least 90% with said SEQ ID NO: 19 or with fragment thereof having at least 5 aminoacids, preferably from 6 to 20 aminoacids, more preferably from 8 to 15 aminoacids.

2. Method according to claim 1, wherein *Brevibacillus laterosporus* is chosen from the group consisting of *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9, Brevibacillus laterosporus PE36, Brevibacillus laterosporus B9, Brevibacillus laterosporus DSM25* (ATCC 64).

3. Method for the detection and/or quantification in a sample of *Brevibacillus laterosporus,* said method comprising or consisting of the detection of at least one marker selected from the group consisting of:
a nucleic acid sequence comprising or consisting of: SEQ ID NO:2 or the complement nucleic acid sequence thereof; a fragment of said SEQ ID NO:2 or complement nucleic acid sequence thereof, said fragment having at least 12 bp, preferably from 15 to 30 bp, more preferably from 18 to 24 bp; or
a nucleic acid sequence having a sequence identity of at least 80%, preferably 90% with said SEQ ID NO: 2, the complement nucleic acid sequence or said fragment;
a surface polypeptide sequence comprising or consisting of: SEQ ID NO: 20, a fragment thereof having at least 5 aminoacids, preferably from 6 to 20 aminoacids, more preferably from 8 to 15 aminoacids;
a polypeptide sequence having a sequence identity of at least 90% with said SEQ ID NO: 20 or with fragment thereof having at least 5 aminoacids, preferably from 6 to 20 aminoacids, more preferably from 8 to 15 aminoacids.

4. Method according to claim 3, wherein *Brevibacillus laterosporus* is *Brevibacillus laterosporus* NCIMB 41419.

5. Method according to anyone of the claims 1-2, said method being carried out by means of PCR and/or Real Time PCR techniques by the use of at least one of the following primer pairs:
5'-GCTTCACACGATCAGCAACC-3' (SEQ ID NO:3)
5'-TGTAGGCGGGCAGCTAAAAA -3' (SEQ ID NO:4);
5'-C AGC TTG GTT CAC TTT ATT CG-3' (SEQ ID NO:5)
5'-TG AAG CAA GCA GGT AGT GAA-3'(SEQ ID NO:6);
5'-CGT TTT TAC TTC TTG TCT TCC TAG-3'(SEQ ID NO:7)
5'-AG GTT GCT GAT CGT GTG A-3'(SEQ ID NO:8);
5'-AGC CTT AGC ACC TGT ATC TTT G-3'(SEQ ID NO:9) 5'-AG GCA GCT ACT GAA GCT GA-3'(SEQ ID NO:10);
5'-CTGCTACTAGTTGATCTAAG-3'(SEQ ID NO:11)
5'-CTGATTGGTAGCTTAGGTA-3'(SEQ ID NO:12);
preferably
5'-CTGCTACTAGTTGATCTAAG-3' (SEQ ID NO:11)
5'-CTGATTGGTAGCTTAGGTA-3'(SEQ ID NO:12)
or
5'-GCTTCACACGATCAGCAACC-3' (SEQ ID NO:3)
5'-TGTAGGCGGGCAGCTAAAAA -3' (SEQ ID NO:4).

6. Method according to any one of claims 3-4, said method being carried out by means of PCR and/or real Time PCR techniques by the use of at least one of the following primer pairs:
5'-CTA TTT ACA CGG GCG TAC G-3'(SEQ ID NO:13)
5'-CAT TCT TTT GAA AGTAGTAAG AAG-3'(SEQ ID NO:14);
5'-TCACCAAGACACAAAGCCCT-3'(SEQ ID NO:15)
5'-CTCTTTGCCGTAGATTCGCG-3'(SEQ ID NO:16);
5'-TCAGGGCATCACGTACACTT-3' (SEQ ID NO:17)
5'-GGGCTTTGTGTCTTGGTGAG-3'(SEQ ID NO:18);
preferably
5'-CTA TTT ACA CGG GCG TAC G-3'(SEQ ID NO:13)
5'-CAT TCT TTT GAA AGTAGTAAG AAG-3'(SEQ ID NO:14)

7. Method for the detection of *Brevibacillus laterosporus* in a sample, said method comprising the method as defined in anyone of the claims 1-2, 5 and the method as defined in anyone of the claims 3-4, 6.

8. Kit for the detection and/or quantification of *Brevibacillus laterosporus* in a sample, said kit comprising or consisting of at least one of the following primer pairs:
5'-GCTTCACACGATCAGCAACC-3' (SEQ ID NO:3)
5'- TGTAGGCGGGCAGCTAAAAA -3'(SEQ ID NO:4);
5'-C AGC TTG GTT CAC TTT ATT CG-3' (SEQ ID NO:5)
5'-TG AAG CAA GCA GGT AGT GAA-3'(SEQ ID NO:6);
5'-CGT TTT TAC TTC TTG TCT TCC TAG-3'(SEQ ID NO:7)
5'-AG GTT GCT GAT CGT GTG A-3'(SEQ ID NO:8);
5'-AGC CTT AGC ACC TGT ATC TTT G-3'(SEQ ID NO:9)
5'-AG GCA GCT ACT GAA GCT GA-3'(SEQ ID NO:10);
5'-CTGCTACTAGTTGATCTAAG-3'(SEQ ID NO:11)
5'-CTGATTGGTAGCTTAGGTA-3'(SEQ ID NO:12);
preferably
5'-CTGCTACTAGTTGATCTAAG-3' (SEQ ID NO:11)
5'-CTGATTGGTAGCTTAGGTA-3'(SEQ ID NO:12) -
or
5'-GCTTCACACGATCAGCAACC-3' (SEQ ID NO:3)
5'-TGTAGGCGGGCAGCTAAAAA -3' (SEQ ID NO:4).
together with suitable reactive agents for the detection and/or quantification.

9. Kit according to claim 8, wherein *Brevibacillus laterosporus* is chosen from the group consisting of *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9, Brevibacillus laterosporus PE36, Brevibacillus laterosporus B9, Brevibacillus laterosporus DSM25* (ATCC 64).

10. Kit for the detection and/or quantification in a sample of *Brevibacillus laterosporus,* said kit comprising or consisting of at least one of the following primer pairs:
5'-CTA TTT ACA CGG GCG TAC G-3'(SEQ ID NO:13)
5'-CAT TCT TTT GAA AGTAGTAAG AAG-3'(SEQ ID NO:14);
5'-TCACCAAGACACAAAGCCCT-3'(SEQ ID NO:15)
5'-CTCTTTGCCGTAGATTCGCG-3'(SEQ ID NO:16);
5'-TCAGGGCATCACGTACACTT-3' (SEQ ID NO:17)
5'-GGGCTTTGTGTCTTGGTGAG-3'(SEQ ID NO:18);
preferably
5'-CTA TTT ACA CGG GCG TAC G-3'(SEQ ID NO:13)
5'-CAT TCT TTT GAA AGTAGTAAG AAG-3'(SEQ ID NO:14)
together with suitable reactive agents for the detection and/or quantification.

11. Kit according to claim 10, wherein *Brevibacillus laterosporus* is *Brevibacillus laterosporus* NCIMB 41419.

12. Kit for the detection and/or quantification in a sample of *Brevibacillus laterosporus,* said kit comprising or consisting of the kit according to claims 8-9 and 10-11.

## Patentansprüche

1. Verfahren zum Nachweis und/oder zur Quantifizierung von *Brevibacillus laterosporus* in einer Probe, wobei das Verfahren den Nachweis und/oder die Quantifizierung von mindestens einem Marker, ausgewählt aus der Gruppe, bestehend aus:
einer Nukleinsäuresequenz, umfassend oder bestehend aus SEQ ID NR.:1 oder der komplementären Nukleinsäuresequenz davon; einem Fragment von der SEQ ID NR.:1 oder komplementären Nukleinsäuresequenz davon, wobei das Fragment mindestens 12 bp, vorzugsweise von 15 bis 30 bp, bevorzugter von 18 bis 24 bp aufweist; einer Nukleinsäuresequenz, die eine Sequenzidentität von mindestens 80 %, vorzugsweise 90 % mit der SEQ ID NR.: 1, komplementären Nukleinsäuresequenz oder dem Fragment aufweist;
einer Oberflächenpolypeptidsequenz, umfassend oder bestehend aus SEQ ID NR.: 19, einem Fragment davon, das mindestens 5 Aminosäuren, vorzugsweise von 6 bis 20 Aminosäuren, bevorzugter von 8 bis 15 Aminosäuren aufweist;
einer Polypeptidsequenz, die eine Sequenzidentität von mindestens 90 % mit der SEQ ID NR.: 19 oder mit einem Fragment davon mit mindestens 5 Aminosäuren, vorzugsweise von 6 bis 20 Aminosäuren, bevorzugter von 8 bis 15 Aminosäuren aufweist,
umfasst oder daraus besteht.

2. Verfahren nach Anspruch 1, wobei *Brevibacillus laterosporus* ausgewählt ist aus der Gruppe, bestehend aus *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus* GI-9, *Brevibacillus laterosporus PE36, Brevibacillus laterosporus B9, Brevibacillus laterosporus DSM25* (ATCC 64).

3. Verfahren zum Nachweis und/oder zur Quantifizierung in einer Probe von *Brevibacillus laterosporus,* wobei das Verfahren den Nachweis von mindestens einem Marker, ausgewählt aus der Gruppe, bestehend aus:
einer Nukleinsäuresequenz, umfassend oder bestehend aus: SEQ ID NR.:2 oder der komplementären Nukleinsäuresequenz davon; einem Fragment von der SEQ ID NR.:2 oder komplementären Nukleinsäuresequenz davon, wobei das Fragment mindestens 12 bp, vorzugsweise von 15 bis 30 bp, bevorzugter von 18 bis 24 bp aufweist; oder
einer Nukleinsäuresequenz, die eine Sequenzidentität von mindestens 80 %, vorzugsweise 90 % mit der SEQ ID NR.: 2, der komplementären Nukleinsäuresequenz oder dem Fragment aufweist;
einer Oberflächenpolypeptidsequenz, umfassend oder bestehend aus: SEQ ID NR.: 20, einem Fragment davon mit mindestens 5 Aminosäuren, vorzugsweise von 6 bis 20 Aminosäuren, bevorzugter von 8 bis 15 Aminosäuren;
einer Polypeptidsequenz, die eine Sequenzidentität von mindestens 90 % mit der SEQ ID NR.: 20 oder mit einem Fragment davon mit mindestens 5 Aminosäuren, vorzugsweise von 6 bis 20 Aminosäuren, bevorzugter von 8 bis 15 Aminosäuren aufweist,
umfasst oder daraus besteht.

4. Verfahren nach Anspruch 3, wobei *Brevibacillus laterosporus Brevibacillus laterosporus* NCIMB 41419 ist.

5. Verfahren nach einem der Ansprüche 1-2, wobei das Verfahren mit Hilfe von PCR - und/oder Echtzeit-PCR-Techniken durch die Verwendung von mindestens einem der nachstehenden Primerpaare ausgeführt wird:
5'-GCTTCACACGATCAGCAACC-3' (SEQ ID NR.:3)
5'-TGTAGGCGGGCAGCTAAAAA -3' (SEQ ID NR.:4);
5'-C AGC TTG GTT CAC TTT ATT CG-3' (SEQ ID NR.:5)
5'-TG AAG CAA GCA GGT AGT GAA-3' (SEQ ID NR.:6);
5'-CGT TTT TAC TTC TTG TCT TCC TAG-3' (SEQ ID NR.:7)
5'-AG GTT GCT GAT CGT GTG A-3' (SEQ ID NR.:8);
5'-AGC CTT AGC ACC TGT ATC TTT G-3' (SEQ ID NR.:9)
5'-AG GCA GCT ACT GAA GCT GA-3' (SEQ ID NR.:10);
5'-CTGCTACTAGTTGATCTAAG-3' (SEQ ID NR.:11)
5'-CTGATTGGTAGCTTAGGTA-3' (SEQ ID NR.:12);
vorzugsweise
5'-CTGCTACTAGTTGATCTAAG-3' (SEQ ID NR.:11)
5'-CTGATTGGTAGCTTAGGTA-3' (SEQ ID NR.:12)
oder
5'-GCTTCACACGATCAGCAACC-3' (SEQ ID NR.:3)
5'-TGTAGGCGGGCAGCTAAAAA -3' (SEQ ID NR.:4).

6. Verfahren nach einem der Ansprüche 3-4, wobei das Verfahren mit Hilfe von PCR- und/oder Echtzeit-PCR-Techniken durch die Verwendung von mindestens einem der nachstehenden Primerpaare ausgeführt wird:
5'-CTA TTT ACA CGG GCG TAC G-3' (SEQ ID NR.:13)
5'-CAT TCT TTT GAA AGTAGTAAG AAG-3' (SEQ ID NR.:14);
5'-TCACCAAGACACAAAGCCCT-3' (SEQ ID NR.:15)
5'-CTCTTTGCCGTAGATTCGCG-3' (SEQ ID NR.:16);
5'-TCAGGGCATCACGTACACTT-3' (SEQ ID NR.:17)
5'-GGGCTTTGTGTCTTGGTGAG-3' (SEQ ID NR.:18);
vorzugsweise
5'-CTA TTT ACA CGG GCG TAC G-3' (SEQ ID NR.:13)
5'-CAT TCT TTT GAA AGTAGTAAG AAG-3' (SEQ ID NR.:14)

7. Verfahren zum Nachweis von *Brevibacillus laterosporus* in einer Probe, wobei das Verfahren das Verfahren, wie in einem der Ansprüche 1-2, 5 definiert, und das Verfahren, wie in einem der Ansprüche 3-4, 6 definiert, umfasst.

8. Kit zum Nachweis und/oder zur Quantifizierung von *Brevibacillus laterosporus* in einer Probe, wobei das Kit mindestens eines der nachstehenden Primerpaare umfasst oder daraus besteht:
5'-GCTTCACACGATCAGCAACC-3' (SEQ ID NR.:3)
5'-TGTAGGCGGGCAGCTAAAAA-3' (SEQ ID NR.:4);
5'-C AGC TTG GTT CAC TTT ATT CG-3' (SEQ ID NR.:5)
5'-TG AAG CAA GCA GGT AGT GAA-3' (SEQ ID NR.:6);
5'-CGT TTT TAC TTC TTG TCT TCC TAG-3' (SEQ ID NR.:7)
5'-AG GTT GCT GAT CGT GTG A-3' (SEQ ID NR.:8);
5'-AGC CTT AGC ACC TGT ATC TTT G-3' (SEQ ID NR.:9)
5'-AG GCA GCT ACT GAA GCT GA-3' (SEQ ID NR.:10);
5'-CTGCTACTAGTTGATCTAAG-3' (SEQ ID NR.:11)
5'-CTGATTGGTAGCTTAGGTA-3' (SEQ ID NR.:12);
vorzugsweise
5'-CTGCTACTAGTTGATCTAAG-3' (SEQ ID NR.:11)
5'-CTGATTGGTAGCTTAGGTA-3'(SEQ ID NR.:12)
oder
5'-GCTTCACACGATCAGCAACC-3' (SEQ ID NR.:3)
5'-TGTAGGCGGGCAGCTAAAAA -3' (SEQ ID NR.:4)
zusammen mit geeigneten reaktiven Mitteln für den Nachweis und/oder die Quantifizierung.

9. Kit nach Anspruch 8, wobei *Brevibacillus laterosporus* ausgewählt ist aus der Gruppe, bestehend aus *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9, Brevibacillus laterosporus PE36, Brevibacillus laterosporus B9, Brevibacillus laterosporus DSM25* (ATCC 64).

10. Kit zum Nachweis und/oder zur Quantifizierung von *Brevibacillus laterosporus* in einer Probe, wobei das Kit mindestens eines der nachstehenden Primerpaare umfasst oder daraus besteht:
5'-CTA TTT ACA CGG GCG TAC G-3' (SEQ ID NR.:13)
5'-CAT TCT TTT GAA AGTAGTAAG AAG-3' (SEQ ID NR.:14);
5'-TCACCAAGACACAAAGCCCT-3' (SEQ ID NR.:15)
5'-CTCTTTGCCGTAGATTCGCG-3' (SEQ ID NR.:16);
5'-TCAGGGCATCACGTACACTT-3' (SEQ ID NR.:17)
5'-GGGCTTTGTGTCTTGGTGAG-3' (SEQ ID NR.:18);
vorzugsweise
5'-CTA TTT ACA CGG GCG TAC G-3' (SEQ ID NR.:13)
5'-CAT TCT TTT GAA AGTAGTAAG AAG-3' (SEQ ID NR.:14)
zusammen mit geeigneten reaktiven Mitteln für den Nachweis und/oder die Quantifizierung.

11. Kit nach Anspruch 10, wobei *Brevibacillus laterosporus Brevibacillus laterosporus* NCIMB 41419 ist.

12. Kit zum Nachweis und/oder zur Quantifizierung von *Brevibacillus laterosporus* in einer Probe, wobei das Kit das Kit nach Ansprüchen 8-9 und 10-11 umfasst oder daraus besteht.

## Revendications

1. Procédé de détection et/ou de quantification de *Brevibacillus laterosporus* dans un échantillon, ledit procédé comprenant ou consistant en la détection et/ou la quantification d'au moins un marqueur choisi dans le groupe constitué par :
une séquence d'acide nucléique comprenant ou consistant en la SEQ ID NO: 1 ou la séquence d'acide nucléique complémentaire de celle-ci ; un fragment de ladite SEQ ID NO: 1 ou une séquence d'acide nucléique complémentaire de celui-ci, ledit fragment ayant au moins 12 bp, de préférence de 15 à 30 bp, de préférence encore de 18 à 24 bp ; une séquence d'acide nucléique ayant une identité de séquence d'au moins 80%, de préférence 90% avec ladite SEQ ID NO: 1, la séquence d'acide nucléique complémentaire ou ledit fragment ;
une séquence polypeptidique de surface comprenant ou consistant en la SEQ ID NO: 19, un fragment de celle-ci ayant au moins 5 acides aminés, de préférence de 6 à 20 acides aminés, de préférence encore de 8 à 15 acides aminés ;
une séquence polypeptidique ayant une identité de séquence d'au moins 90% avec ladite SEQ ID NO: 19 ou avec un fragment de celle-ci ayant au moins 5 acides aminés, de préférence de 6 à 20 acides aminés, de préférence encore de 8 à 15 acides aminés.

2. Procédé selon la revendication 1, dans lequel Brevibacillus laterosporus est choisi dans le groupe consistant en *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9, Brevibacillus laterosporus PE36, Brevibacillus laterosporus B9, Brevibacillus laterosporus DSM25* (ATCC 64).

3. Procédé de détection et/ou de quantification dans un échantillon de *Brevibacillus laterosporus,* ledit procédé comprenant ou consistant en la détection d'au moins un marqueur choisi dans le groupe constitué par :
une séquence d'acide nucléique comprenant ou consistant en : la SEQ ID NO: 2 ou la séquence d'acide nucléique complémentaire de celle-ci ; un fragment de ladite SEQ ID NO: 2 ou une séquence d'acide nucléique complémentaire de celui-ci, ledit fragment ayant au moins 12 bp, de préférence de 15 à 30 bp, de préférence encore de 18 à 24 bp ; ou
une séquence d'acide nucléique ayant une identité de séquence d'au moins 80%, de préférence de 90% avec ladite SEQ ID NO: 2, la séquence d'acide nucléique complémentaire ou ledit fragment ;
une séquence polypeptidique de surface comprenant ou consistant en : la SEQ ID NO: 20, un fragment de celle-ci ayant au moins 5 acides aminés, de préférence de 6 à 20 acides aminés, de préférence encore de 8 à 15 acides aminés ;
une séquence polypeptidique ayant une identité de séquence d'au moins 90% avec ladite SEQ ID NO: 20 ou avec un fragment de celle-ci ayant au moins 5 acides aminés, de préférence de 6 à 20 acides aminés, de préférence encore de 8 à 15 acides aminés.

4. Procédé selon la revendication 3, dans lequel *Brevibacillus laterosporus* est *Brevibacillus laterosporus* NCIMB 41419.

5. Procédé selon l'une quelconque des revendications 1 et 2, ledit procédé étant effectué au moyen de techniques de PCR et/ou de PCR en temps réel par l'utilisation d'au moins l'une des paires d'amorces suivantes :
5'-GCTTCACACGATCAGCAACC-3' (SEQ ID NO: 3)
5'-TGTAGGCGGGCAGCTAAAAA-3' (SEQ ID NO: 4) ;
5'-C AGC TTG GTT CAC TTT ATT CG-3' (SEQ ID NO: 5)
5'-TG AAG CAA GCA GGT AGT GAA-3' (SEQ ID NO: 6) ;
5'-CGT TTT TAC TTC TTG TCT TCC TAG-3' (SEQ ID NO: 7)
5'-AG GTT GCT GAT CGT GTG A-3' (SEQ ID NO: 8) ;
5'-AGC CTT AGC ACC TGT ATC TTT G-3' (SEQ ID NO: 9)
5'-AG GCA GCT ACT GAA GCT GA-3' (SEQ ID NO: 10) ;
5'-CTGCTACTAGTTGATCTAAG-3' (SEQ ID NO: 11)
5'-CTGATTGGTAGCTTAGGTA-3' (SEQ ID NO: 12);
de préférence
5'-CTGCTACTAGTTGATCTAAG-3' (SEQ ID NO: 11)
5'-CTGATTGGTAGCTTAGGTA-3' (SEQ ID NO: 12);
ou
5'-GCTTCACACGATCAGCAACC-3' (SEQ ID NO: 3)
5'-TGTAGGCGGGCAGCTAAAAA-3' (SEQ ID NO: 4).

6. Procédé selon l'une quelconque des revendications 3 à 4, ledit procédé étant réalisé au moyen de techniques de PCR et/ou de PCR en temps réel par l'utilisation d'au moins l'une des paires d'amorces suivantes :
5'-CTA TTT ACA CGG GCG TAC G-3' (SEQ ID NO: 13) 5'-CAT TCT TTT GAA AGTAGTAAG AAG-3' (SEQ ID NO: 14) ;
5'-TCACCAAGACACAAAGCCCT-3' (SEQ ID NO: 15) 5'-CTCTTTGCCGTAGATTCGCG-3' (SEQ ID NO: 16) ;
5'-TCAGGGCATCACGTACACTT-3' (SEQ ID NO: 17) 5'-GGGCTTTGTGTCTTGGTGAG-3' (SEQ ID NO: 18) ;
de préférence
5'-CTA TTT ACA CGG GCG TAC G-3' (SEQ ID NO: 13) 5'-CAT TCT TTT GAA AGTAGTAAG AAG-3' (SEQ ID NO: 14).

7. Procédé de détection de *Brevibacillus laterosporus* dans un échantillon, ledit procédé comprenant le procédé tel que défini dans l'une quelconque des revendications 1-2, 5 et le procédé tel que défini dans l'une quelconque des revendications 3-4, 6.

8. Kit pour la détection et/ou la quantification de *Brevibacillus laterosporus* dans un échantillon, ledit kit comprenant ou consistant en au moins une des paires d'amorces suivantes :
5'-GCTTCACACGATCAGCAACC-3' (SEQ ID NO: 3)
5'-TGTAGGCGGGCAGCTAAAAA-3' (SEQ ID NO: 4) ;
5'-C AGC TTG GTT CAC TTT ATT CG-3' (SEQ ID NO: 5)
5'-TG AAG CAA GCA GGT AGT GAA-3' (SEQ ID NO: 6) ;
5'-CGT TTT TAC TTC TTG TCT TCC TAG-3' (SEQ ID NO: 7)
5'-AG GTT GCT GAT CGT GTG A-3' (SEQ ID NO: 8) ;
5'-AGC CTT AGC ACC TGT ATC TTT G-3' (SEQ ID NO: 9)
5'-AG GCA GCT ACT GAA GCT GA-3' (SEQ ID NO: 10) ;
5'-CTGCTACTAGTTGATCTAAG-3' (SEQ ID NO: 11)
5'-CTGATTGGTAGCTTAGGTA-3' (SEQ ID NO: 12);
de préférence
5'-CTGCTACTAGTTGATCTAAG-3' (SEQ ID NO: 11)
5'-CTGATTGGTAGCTTAGGTA-3' (SEQ ID NO: 12);
ou
5'-GCTTCACACGATCAGCAACC-3' (SEQ ID NO: 3)
5'-TGTAGGCGGGCAGCTAAAAA-3' (SEQ ID NO: 4),
avec des agents réactifs appropriés pour la détection et/ou la quantification.

9. Kit selon la revendication 8, dans lequel *Brevibacillus laterosporus* est choisi dans le groupe consistant en *Brevibacillus laterosporus* ATCC9141, *Brevibacillus laterosporus* ATCC6456, *Brevibacillus laterosporus* BOD ATCC 55122, *Brevibacillus laterosporus* NCIMB 41419, *Brevibacillus laterosporus GI-9, Brevibacillus laterosporus PE36, Brevibacillus laterosporus B9, Brevibacillus laterosporus* DSM25 (ATCC 64).

10. Kit pour la détection et/ou la quantification dans un échantillon de *Brevibacillus laterosporus,* ledit kit comprenant ou consistant en au moins une des paires d'amorces suivantes :
5'-CTA TTT ACA CGG GCG TAC G-3' (SEQ ID NO: 13)
5'-CAT TCT TTT GAA AGTAGTAAG AAG-3' (SEQ ID NO: 14) ;
5'-TCACCAAGACACAAAGCCCT-3' (SEQ ID NO: 15)
5'-CTCTTTGCCGTAGATTCGCG-3' (SEQ ID NO: 16) ;
5'-TCAGGGCATCACGTACACTT-3' (SEQ ID NO: 17)
5'-GGGCTTTGTGTCTTGGTGAG-3' (SEQ ID NO: 18) ;
de préférence
5'-CTA TTT ACA CGG GCG TAC G-3' (SEQ ID NO: 13)
5'-CAT TCT TTT GAA AGTAGTAAG AAG-3' (SEQ ID NO: 14).
avec des agents réactifs appropriés pour la détection et/ou la quantification.

11. Kit selon la revendication 10, dans lequel *Brevibacillus laterosporus* est *Brevibacillus laterosporus* NCIMB 41419.

12. Kit pour la détection et/ou la quantification dans un échantillon de *Brevibacillus laterosporus,* ledit kit comprenant ou consistant en le kit selon les revendications 8-9 et 10-11.


### SEQUENCE LISTING

<110> Universita degli Studi di Sassari
<120> Markers for the detection of Brevibacillus laterosporus and
   related methods and kits
<130> PCT30232
<150> RM2015A000084
   <151> 2015-02-26
<160> 52
<170> PatentIn version 3.5
<210> 1
   <211> 771
   <212> DNA
   <213> Brevibacillus laterosporus
<400> 1
   ttatttaaat tgatctgcta ctagttgatc taagctattc tttgcatcct tttcagcctt      60
   agcacctgta tctttgatcg aatccttcgc ttgtttctcc aataatttag ctgattcgtc     120
   aactactttt tggatttcag cgactttttc gtcgataaag cgttttgttt cctctttaga     180
   gctagcttct ttctcacgaa tcaaaccatt aattgtcttt ttagcatcgt taatcgcact     240
   agttagctct tgttttgctt tgtcttgcgc actggtaaca ttcgttttta cttcttgtct     300
   tcctagttct ccagcttggt tcactttatt cgtaagatcg actatcgaat tgacttttgt     360
   ttgattggct gctttagtaa tttcctgctt taatccttct accttctggt tgtattggtt     420
   ctgcatattg cccttgattt cattcgtttt gcctctcaaa gcattcaaat aatcgttttt     480
   agctgccgtt actgctgcaa cagcacgatt tgcttcttca ctacctgctt gcttcacacg     540
   atcagcaacc tgattcttta ttttattaag ttcagcttca gtagctgcct ttttttccgt     600
   tccatactta agcgcttcag ccgtaataga cgccgatgaa gcagcaaatt gtttatcata     660
   ccaatttttt agctgcccgc ctacatcaac cgccgcaaat gcagtaccta agctaccaat     720
   cagaccaact gccaacacac taatcacgat tttacttttt acttttttca a              771
<210> 2
   <211> 420
   <212> DNA
   <213> Brevibacillus laterosporus
<400> 2
   ctatttacac gggcgtacga tttcttttgt taacactttc agggcatcac gtacactttt      60
   agcgaagacc aaaggcttct caatcttttc atagtggata taccatagat tagggttggt     120
   aaatagccag tggttatgaa gggcagttac ctttatgcca tgttctctaa gtcttgaaat     180
   atacggattg atctcctcag tgagaatgac cgtctcacca agacacaaag ccctaccatt     240
   cttcttacta ctttcaaaag aatgaaattg tgggatggtt aagggagatt tcgaccttct     300
   gcctaagata gtaggtttta tgttggtacg gagggtctgg gcagtacaga caccattcac     360
   gactgtgggc ttggcgttaa gaatttccgc gaatctacgg caaagagggc taattttcat     420
<210> 3
   <211> 20
   <212> DNA
   <213> Artificial Sequence
<220>
   <223> Brevibacillus laterosporus SC-CSPB complex forward primer
<400> 3
   gcttcacacg atcagcaacc 20
<210> 4
   <211> 20
   <212> DNA
   <213> Artificial Sequence
<220>
   <223> Brevibacillus laterosporus SC-CSPB complex reverse primer
<400> 4
   tgtaggcggg cagctaaaaa 20
<210> 5
   <211> 21
   <212> DNA
   <213> Artificial Sequence
<220>
   <223> Brevibacillus laterosporus SC-CSPB complex forward primer
<400> 5
   cagcttggtt cactttattc g 21
<210> 6
   <211> 20
   <212> DNA
   <213> Artificial Sequence
<220>
   <223> Brevibacillus laterosporus SC-CSPB complex reverse primer
<400> 6
   tgaagcaagc aggtagtgaa 20
<210> 7
   <211> 24
   <212> DNA
   <213> Artificial Sequence
<220>
   <223> Brevibacillus laterosporus SC-CSPB complex forward primer
<400> 7
   cgtttttact tcttgtcttc ctag 24
<210> 8
   <211> 18
   <212> DNA
   <213> Artificial Sequence
<220>
   <223> Brevibacillus laterosporus SC-CSPB complex reverse primer
<400> 8
   aggttgctga tcgtgtga 18
<210> 9
   <211> 22
   <212> DNA
   <213> Artificial Sequence
<220>
   <223> Brevibacillus laterosporus SC-CSPB complex reverse primer
<400> 9
   agccttagca cctgtatctt tg 22
<210> 10
   <211> 19
   <212> DNA
   <213> Artificial Sequence
<220>
   <223> Brevibacillus laterosporus SC-CSPB complex reverse primer
<400> 10
   aggcagctac tgaagctga 19
<210> 11
   <211> 20
   <212> DNA
   <213> Artificial Sequence
<220>
   <223> Brevibacillus laterosporus SC-CSPB complex forward primer
<400> 11
   ctgctactag ttgatctaag 20
<210> 12
   <211> 19
   <212> DNA
   <213> Artificial sequence
<220>
   <223> Brevibacillus laterosporus SC-CSPB complex reverse primer
<400> 12
   ctgattggta gcttaggta 19
<210> 13
   <211> 19
   <212> DNA
   <213> Artificial Sequence
<220>
   <223> Brevibacillus laterosporus SC-CSPB complex forward primer
<400> 13
   ctatttacac gggcgtacg 19
<210> 14
   <211> 24
   <212> DNA
   <213> Artificial sequence
<220>
   <223> Brevibacillus laterosporus SC-CSPB complex reverse primer
<400> 14
   cattcttttg aaagtagtaa gaag 24
<210> 15
   <211> 20
   <212> DNA
   <213> Artificial Sequence
<220>
   <223> Brevibacillus laterosporus SC-CSPB complex forward primer
<400> 15
   tcaccaagac acaaagccct 20
<210> 16
   <211> 20
   <212> DNA
   <213> Artificial Sequence
<220>
   <223> Brevibacillus laterosporus SC-CSPB complex reverse primer
<400> 16
   ctctttgccg tagattcgcg 20
<210> 17
   <211> 20
   <212> DNA
   <213> Artificial Sequence
<220>
   <223> Brevibacillus laterosporus SC-CSPB complex forward primer
<400> 17
   tcagggcatc acgtacactt 20
<210> 18
   <211> 20
   <212> DNA
   <213> Artificial sequence
<220>
   <223> Brevibacillus laterosporus SC-CSPB complex reverse primer
<400> 18
   gggctttgtg tcttggtgag 20
<210> 19
   <211> 256
   <212> PRT
   <213> Brevibacillus laterosporus
<400> 19
   Met Lys Lys Val Lys Ser Lys Ile Val Ile Ser Val Leu Ala Val Gly
   1               5                   10                  15
   Leu Ile Gly Ser Leu Gly Thr Ala Phe Ala Ala Val Asp Val Gly Gly
               20                  25                  30
   Gln Leu Lys Asn Trp Tyr Asp Lys Gln Phe Ala Ala Ser Ser Ala Ser
           35                  40                  45
   Ile Thr Ala Glu Ala Leu Lys Tyr Gly Thr Glu Lys Lys Ala Ala Thr
       50                  55                  60
   Glu Ala Glu Leu Asn Lys Ile Lys Asn Gln Val Ala Asp Arg Val Lys
   65                  70                  75                  80
   Gln Ala Gly Ser Glu Glu Ala Asn Arg Ala Val Ala Ala Val Thr Ala
                   85                  90                  95
   Ala Lys Asn Asp Tyr Leu Asn Ala Leu Arg Gly Lys Thr Asn Glu Ile
               100                 105                 110
   Lys Gly Asn Met Gln Asn Gln Tyr Asn Gln Lys Val Glu Gly Leu Lys
           115                 120                 125
   Gln Glu Ile Thr Lys Ala Ala Asn Gln Thr Lys Val Asn Ser Ile Val
       130                 135                 140
   Asp Leu Thr Asn Lys Val Asn Gln Ala Gly Glu Leu Gly Arg Gln Glu
   145                 150                 155                 160
   Val Lys Thr Asn Val Thr Ser Ala Gln Asp Lys Ala Lys Gln Glu Leu
                   165                 170                 175
   Thr Ser Ala Ile Asn Asp Ala Lys Lys Thr Ile Asn Gly Leu Ile Arg
               180                 185                 190
   Glu Lys Glu Ala Ser Ser Lys Glu Glu Thr Lys Arg Phe Ile Asp Glu
           195                 200                 205
   Lys Val Ala Glu Ile Gln Lys Val Val Asp Glu Ser Ala Lys Leu Leu
       210                 215                 220
   Glu Lys Gln Ala Lys Asp Ser Ile Lys Asp Thr Gly Ala Lys Ala Glu
   225                 230                 235                 240
   Lys Asp Ala Lys Asn Ser Leu Asp Gln Leu Val Ala Asp Gln Phe Lys
                   245                 250                 255
<210> 20
   <211> 139
   <212> PRT
   <213> Brevibacillus laterosporus
<400> 20
   Met Lys Ile Ser Pro Leu Cys Arg Arg Phe Ala Glu Ile Leu Asn Ala
   1               5                   10                  15
   Lys Pro Thr Val Val Asn Gly Val Cys Thr Ala Gln Thr Leu Arg Thr
               20                  25                  30
   Asn Ile Lys Pro Thr Ile Leu Gly Arg Arg Ser Lys Ser Pro Leu Thr
           35                  40                  45
   Ile Pro Gln Phe His Ser Phe Glu Ser Ser Lys Lys Asn Gly Arg Ala
       50                  55                  60
   Leu Cys Leu Gly Glu Thr Val Ile Leu Thr Glu Glu Ile Asn Pro Tyr
   65                  70                  75                  80
   Ile Ser Arg Leu Arg Glu His Gly Ile Lys Val Thr Ala Leu His Asn
                   85                  90                  95
   His Trp Leu Phe Thr Asn Pro Asn Leu Trp Tyr Ile His Tyr Glu Lys
               100                 105                 110
   Ile Glu Lys Pro Leu Val Phe Ala Lys Ser Val Arg Asp Ala Leu Lys
           115                 120                 125
   Val Leu Thr Lys Glu Ile Val Arg Pro Cys Lys
       130                 135
<210> 21
   <211> 771
   <212> DNA
   <213> Brevibacillus laterosporus
<400> 21
   ttgaaaaaag taaaaagtaa aatcgtgatt agtgtgttgg cagttggtct gattggtagc 60
   ttaggtactg catttgcggc ggttgatgta ggcgggcagc taaaaaattg gtatgataaa 120
   caatttgctg cttcatcggc gtctattacg gctgaagcgc ttaagtatgg aacggaaaaa 180
   aaggcagcta ctgaagctga acttaataaa ataaagaatc aggttgctga tcgtgtgaag 240
   caagcaggta gtgaagaagc aaatcgtgct gttgcagcag taacggcagc taaaaacgat 300
   tatttgaatg ctttgagagg caaaacgaat gaaatcaagg gcaatatgca gaaccaatac 360
   aaccagaagg tagaaggatt aaagcaggaa attactaaag cagccaatca aacaaaagtc 420
   aattcgatag tcgatcttac gaataaagtg aaccaagctg gagaactagg aagacaagaa 480
   gtaaaaacga atgttaccag tgcgcaagac aaagcaaaac aagagctaac tagtgcgatt 540
   aacgatgcta aaaagacaat taatggtttg attcgtgaga aagaagctag ctctaaagag 600
   gaaacaaaac gctttatcga cgaaaaagtc gctgaaatcc aaaaagtagt tgacgaatca 660
   gctaaattat tggagaaaca agcgaaggat tcgatcaaag atacaggtgc taaggctgaa 720
   aaggatgcaa agaatagctt agatcaacta gtagcagatc aatttaaata a 771
<210> 22
   <211> 771
   <212> DNA
   <213> Brevibacillus laterosporus
<400> 22
   ttatttaaat tgatccgcta ctagttgatc taagctattc tttgcatcct tttcagcctt 60
   agcacctgta tctttgatcg aatccttcgc ttgtttctcc aataatttag ctgattcgtc 120
   aactactttt tggatttcag cgactttttc gtcgataaag cgttttgttt cctctttaga 180
   gctagcttct ttctcacgaa tcaaaccatt aattgtcttt ttagcatcgt taatcgcact 240
   agttagctct tgttttgctt tgtcttgcgc actggtaaca ttcgttttta cttcttgtct 300
   tcctagttct ccagcttggt tcactttatt cgtaagatcg actatcgaat tgacttttgt 360
   ttgattggct gctttagtaa tttcctgctt taatccttct accttctggt tgtattggtt 420
   ctgcatattg cccttgattt cattcgtttt gcctctcaaa gcattcaaat aatcgttttt 480
   agctgccgtt actgctgcaa cagcacgatt tgcttcttca ctacctgctt gcttcacacg 540
   atcagcaacc tgattcttta ttttattaag ttcagcttca gtagctgcct ttttttccgt 600
   tccatactta agcgcttcag ccgtaataga cgccgatgaa gcagcaaatt gtttatcata 660
   ccaatttttt agctgcccgc ctacatcaac cgccgcaaat gcagtaccta agctaccaat 720
   cagaccaact gccaacacac taatcacgat tttacttttt acttttttca a 771
<210> 23
   <211> 771
   <212> DNA
   <213> Brevibacillus laterosporus
<400> 23
   ttgaaaaaag taaaaagtaa aatcgtgatt agtgtgttgg cagttggtct gattggtagc      60
   ttaggtactg catttgcggc ggttgatgta ggcgggcagc taaaaaattg gtatgataaa     120
   caatttgctg cttcatcggc gtctattacg gctgaagcgc ttaagtatgg aacggaaaaa     180
   aaggcagcta ctgaagctga acttaataaa ataaagaatc aggttgctga tcgtgtgaag     240
   caagcaggta gtgaagaagc aaatcgtgct gttgcagcag taacggcagc taaaaacgat     300
   tatttgaatg ctttgagagg caaaacgaat gaaatcaagg gcaatatgca gaaccaatac     360
   aaccagaagg tagaaggatt aaagcaggaa attactaaag cagccaatca aacaaaagtc     420
   aattcgatag tcgatcttac gaataaagtg aaccaagctg gagaactagg aagacaagaa     480
   gtaaaaacga atgttaccag tgcgcaagac aaagcaaaac aagagctaac tagtgcgatt     540
   aacgatgcta aaaagacaat taatggtttg attcgtgaga aagaagctag ctctaaagag     600
   gaaacaaaac gctttatcga cgaaaaagtc gctgaaatcc aaaaagtagt tgacgaatca     660
   gctaaattat tggagaaaca agcgaaggat tcgatcaaag atacaggtgc taaggctgaa     720
   aaggatgcaa agaatagctt agatcaacta gtagcggatc aatttaaata a              771
<210> 24
   <211> 771
   <212> DNA
   <213> Brevibacillus laterosporus
<400> 24
   ttatttaaat tgatctgcta ctagttgatc taagctattt tttgcatcct tttcagcctt      60
   agcacctgta tctttgatcg aatccttcgc ttgtttctcc aataatttag ctgattcgtc     120
   aactactttt tggatttcag cgactttttc gtcgataaag cgttttgttt cctctttaga     180
   gctagcttct ttctcacgaa tcaaaccatt aattgtcttt ttagcatcgt taatcgcact     240
   agttagctct tgttttgctt tgtcttgcgc actggtaaca ttcgttttta cttcttgtct     300
   tcctagctct ccagcttggt tcactttatt cgtaagatcg actatcgaat tgacttttgt     360
   ttgattggct gctttagtaa tttcctgctt taatccttct accttctggt tgtattggtt     420
   ctgcatattg cccttgattt cattcgtttt gcctctcaaa gcattcaaat aatcgttttt     480
   agctgccgtt actgctgcaa cagcacgatt tgcttcttca ctacctgctt gcttcacacg     540
   atcagcaacc tgattcttta ttttattaag ttcagcttca gtagctgcct ttttttccgt     600
   tccatactta agcgcttcag ccgtaataga cgccgatgaa gcagcaaatt gtttatcata     660
   ccaatttttt agctgcccgc ctacatcaac cgccgcaaat gcagtaccta agctaccaat     720
   cagaccaact gccaacacac taatcacgat tttacttttt acttttttca a              771
<210> 25
   <211> 771
   <212> DNA
   <213> Brevibacillus laterosporus
<400> 25
   ttgaaaaaag taaaaagtaa aatcgtgatt agtgtgttgg cagttggtct gattggtagc      60
   ttaggtactg catttgcggc ggttgatgta ggcgggcagc taaaaaattg gtatgataaa     120
   caatttgctg cttcatcggc gtctattacg gctgaagcgc ttaagtatgg aacggaaaaa     180
   aaggcagcta ctgaagctga acttaataaa ataaagaatc aggttgctga tcgtgtgaag     240
   caagcaggta gtgaagaagc aaatcgtgct gttgcagcag taacggcagc taaaaacgat     300
   tatttgaatg ctttgagagg caaaacgaat gaaatcaagg gcaatatgca gaaccaatac     360
   aaccagaagg tagaaggatt aaagcaggaa attactaaag cagccaatca aacaaaagtc     420
   aattcgatag tcgatcttac gaataaagtg aaccaagctg gagagctagg aagacaagaa     480
   gtaaaaacga atgttaccag tgcgcaagac aaagcaaaac aagagctaac tagtgcgatt     540
   aacgatgcta aaaagacaat taatggtttg attcgtgaga aagaagctag ctctaaagag     600
   gaaacaaaac gctttatcga cgaaaaagtc gctgaaatcc aaaaagtagt tgacgaatca     660
   gctaaattat tggagaaaca agcgaaggat tcgatcaaag atacaggtgc taaggctgaa     720
   aaggatgcaa aaaatagctt agatcaacta gtagcagatc aatttaaata a              771
<210> 26
   <211> 771
   <212> DNA
   <213> Brevibacillus laterosporus
<400> 26
   ttatttaaat tgatctgcta ctagctgatc taagctattt tttgcatcct tttcagcctt      60
   agcacctgta tctttgatag catccttcac ttgtttctcc aataatttag ctgactcgtc     120
   aactactttt tggatttcag ctaccttttc gtcaataaag cgttttgttt cctctttcga     180
   gcttgcttcc ttttcacgaa ttaaaccatt aattgtcttc ttagcatcgt taatcgcact     240
   agttaattct tgttttgctt tatcttgtgc actggtaaca ttcgttctta cttcttgttt     300
   acctagttct ccagcatggt tcactttatt cgtaagatca attaccgaat tgacctttgt     360
   atgattagct gctttgttaa tttcctgctt taatccttcg accttctgat tgtattggtt     420
   ctgcatattg cccttgattt cgttcgtttt gcctctcaaa gcattcaaat aatcgttttt     480
   agcagccgtt actgctgcaa cagcacgatt tgcttcttca ctacctgctt gcttcacacg     540
   gtcagcaacc tgattcttta ttttattaag ctcagcttca gtaactgcct ttttttcgtt     600
   tccatactta accgtttctg tcgtaataga cgccgctgaa gcagcgaatt gtttatcata     660
   ccaatttttt aactgcccgc ctacatcaac cgccgcaaat gcagtaccta agctaccaat     720
   cagaccaact gccaacacac taatcacgat ttactttttt acttttttca a              771
<210> 27
   <211> 771
   <212> DNA
   <213> Brevibacillus laterosporus
<400> 27
   ttgaaaaaag taaaaagtaa aatcgtgatt agtgtgttgg cagttggtct gattggtagc      60
   ttaggtactg catttgcggc ggttgatgta ggcgggcagt taaaaaattg gtatgataaa     120
   caattcgctg cttcagcggc gtctattacg acagaaacgg ttaagtatgg aaacgaaaaa     180
   aaggcagtta ctgaagctga gcttaataaa ataaagaatc aggttgctga ccgtgtgaag     240
   caagcaggta gtgaagaagc aaatcgtgct gttgcagcag taacggctgc taaaaacgat     300
   tatttgaatg ctttgagagg caaaacgaac gaaatcaagg gcaatatgca gaaccaatac     360
   aatcagaagg tcgaaggatt aaagcaggaa attaacaaag cagctaatca tacaaaggtc     420
   aattcggtaa ttgatcttac gaataaagtg aaccatgctg gagaactagg taaacaagaa     480
   gtaagaacga atgttaccag tgcacaagat aaagcaaaac aagaattaac tagtgcgatt     540
   aacgatgcta agaagacaat taatggttta attcgtgaaa aggaagcaag ctcgaaagag     600
   gaaacaaaac gctttattga cgaaaaggta gctgaaatcc aaaaagtagt tgacgagtca     660
   gctaaattat tggagaaaca agtgaaggat gctatcaaag atacaggtgc taaggctgaa     720
   aaggatgcaa aaaatagctt agatcagcta gtagcagatc aatttaaata a              771
<210> 28
   <211> 771
   <212> DNA
   <213> Brevibacillus laterosporus
<400> 28
   ttatttaaat tgatcggcta ctagttgatc caagctattc tttgcatcct tttcagcctt      60
   agcacctgta tctttaatag catccttcac ttgtttctcc aataatttag cggattcgtc     120
   aactactttt tggatttcag caactttttc gtcaataaag cgttttgttt cctctttaga     180
   gctagcttct ttctcgcgaa tcaaaccatt aatcgtattc ttagcatcgt taatcgcact     240
   ggttagttct tgctttgctt tgtcttgtgc actagtaacg tttgttctta cttcttgttt     300
   tcctgcttct ccagcgtggt tcactttatt tgtaagatcg acgatcgaat tgacttttgt     360
   atgattggct gctttgttaa tttcctgctt taatccctct accttctggt tgtattggtt     420
   ctgcatattg cccttgattt cgttcgtttt gcctctcaaa gcattcaaat aatcgttttt     480
   agcagctgtt actgaagcga cggcacgatt tgcttcctcg ctacccgctt gcttaacacg     540
   gtcagcaacc tgattcttta gcttattaat ctcaccttct gtaattgcct ttttctcgtt     600
   tccgtaatta accgcttctt tcgtaataga cgatgctgaa gcagcgaatt gtttatcata     660
   ccaattttta agctggccac ctacatcaac ggccgcaaat gcagtaccta agctaccaat     720
   cagaccaact gctaacacac caatcacgat tttacttttt acttttttca a              771
<210> 29
   <211> 771
   <212> DNA
   <213> Brevibacillus laterosporus
<400> 29
   ttgaaaaaag taaaaagtaa aatcgtgatt ggtgtgttag cagttggtct gattggtagc      60
   ttaggtactg catttgcggc cgttgatgta ggtggccagc ttaaaaattg gtatgataaa     120
   caattcgctg cttcagcatc gtctattacg aaagaagcgg ttaattacgg aaacgagaaa     180
   aaggcaatta cagaaggtga gattaataag ctaaagaatc aggttgctga ccgtgttaag     240
   caagcgggta gcgaggaagc aaatcgtgcc gtcgcttcag taacagctgc taaaaacgat     300
   tatttgaatg ctttgagagg caaaacgaac gaaatcaagg gcaatatgca gaaccaatac     360
   aaccagaagg tagagggatt aaagcaggaa attaacaaag cagccaatca tacaaaagtc     420
   aattcgatcg tcgatcttac aaataaagtg aaccacgctg gagaagcagg aaaacaagaa     480
   gtaagaacaa acgttactag tgcacaagac aaagcaaagc aagaactaac cagtgcgatt     540
   aacgatgcta agaatacgat taatggtttg attcgcgaga aagaagctag ctctaaagag     600
   gaaacaaaac gctttattga cgaaaaagtt gctgaaatcc aaaaagtagt tgacgaatcc     660
   gctaaattat tggagaaaca agtgaaggat gctattaaag atacaggtgc taaggctgaa     720
   aaggatgcaa agaatagctt ggatcaacta gtagccgatc aatttaaata a              771
<210> 30
   <211> 780
   <212> DNA
   <213> Brevibacillus laterosporus
<400> 30
   ttatttaaat tgatctgcta ctagttgatc taagctattc tttgcatcct tttcagcctt      60
   agcacctgta tctttgatcg aatccttcgc ttgtttctcc aataatttag ctgattcgtc 120
   aactactttt tggatttcag cgactttttc gtcgataaag cgttttgttt cctctttaga 180
   gctagcttct ttctcacgaa tcaaaccatt aattgtcttt ttagcatcgt taatcgcact 240
   agttagctct tgttttgctt tgtcttgcgc actggtaaca ttcgttttta cttcttgtct 300
   tcctagttct ccagcttggt tcactttatt cgtaagatcg actatcgaat tgacttttgt 360
   ttgattggct gctttagtaa tttcctgctt taatccttct accttctggt tgtattggtt 420
   ctgcatattg cccttgattt cattcgtttt gcctctcaaa gcattcaaat aatcgttttt 480
   agctgccgtt actgctgcaa cagcacgatt tgcttcttca ctacctgctt gcttcacacg 540
   atcagcaacc tgattcttta ttttattaag ttcagcttca gtagctgcct ttttttccgt 600
   tccatactta agcgcttcag ccgtaataga cgccgatgaa gcagcaaatt gtttatcata 660
   ccaatttttt agctgcccgc ctacatcaac cgccgcaaat gcagtaccta agctaccaat 720
   cagaccaact gccaacacac taatcacgat tttacttttt acttttttca acctcttcat 780
<210> 31
   <211> 780
   <212> DNA
   <213> Brevibacillus laterosporus
<400> 31
   atgaagaggt tgaaaaaagt aaaaagtaaa atcgtgatta gtgtgttggc agttggtctg 60
   attggtagct taggtactgc atttgcggcg gttgatgtag gcgggcagct aaaaaattgg 120
   tatgataaac aatttgctgc ttcatcggcg tctattacgg ctgaagcgct taagtatgga 180
   acggaaaaaa aggcagctac tgaagctgaa cttaataaaa taaagaatca ggttgctgat 240
   cgtgtgaagc aagcaggtag tgaagaagca aatcgtgctg ttgcagcagt aacggcagct 300
   aaaaacgatt atttgaatgc tttgagaggc aaaacgaatg aaatcaaggg caatatgcag 360
   aaccaataca accagaaggt agaaggatta aagcaggaaa ttactaaagc agccaatcaa 420
   acaaaagtca attcgatagt cgatcttacg aataaagtga accaagctgg agaactagga 480
   agacaagaag taaaaacgaa tgttaccagt gcgcaagaca aagcaaaaca agagctaact 540
   agtgcgatta acgatgctaa aaagacaatt aatggtttga ttcgtgagaa agaagctagc 600
   tctaaagagg aaacaaaacg ctttatcgac gaaaaagtcg ctgaaatcca aaaagtagtt 660
   gacgaatcag ctaaattatt ggagaaacaa gcgaaggatt cgatcaaaga tacaggtgct 720
   aaggctgaaa aggatgcaaa gaatagctta gatcaactag tagcagatca atttaaataa 780
<210> 32
   <211> 420
   <212> DNA
   <213> Brevibacillus laterosporus
<400> 32
   atgaaaatta gccctctttg ccgtagattc gcggaaattc ttaacgccaa gcccacagtc 60
   gtgaatggtg tctgtactgc ccagaccctc cgtaccaaca taaaacctac tatcttaggc 120
   agaaggtcga aatctccctt aaccatccca caatttcatt cttttgaaag tagtaagaag 180
   aatggtaggg ctttgtgtct tggtgagacg gtcattctca ctgaggagat caatccgtat 240
   atttcaagac ttagagaaca tggcataaag gtaactgccc ttcataacca ctggctattt 300
   accaacccta atctatggta tatccactat gaaaagattg agaagccttt ggtcttcgct 360
   aaaagtgtac gtgatgccct gaaagtgtta acaaaagaaa tcgtacgccc gtgtaaatag 420
<210> 33
   <211> 420
   <212> DNA
   <213> Brevibacillus laterosporus
<400> 33
   ctatctacac gggcgtacga tttcttttgt taacactttc agggcatcac gtacactttt 60
   agcgaagacc aaaggtttct caatcttttc atagtggata taccatagat tagggttggt 120
   aaatagccag tggttatgaa gggcagttac ctttatgcca tgttctcgaa gtcttgaaat 180
   atacggattg atctcctcag tgagaatgac cgtctcacca agacacaaag ccctaccatt 240
   cttcttaatg ctttcaaaag aatgaaattg tgggatggtt aagggagatt tcgaccttct 300
   gcctaagata gtaggtttta tgttggtacg gagggtctgg gcagtacaga caccattcac 360
   gactgtgggc ttggcgttaa gaatttccgc gaatctacgg caaagagggc taattttcat 420
<210> 34
   <211> 420
   <212> DNA
   <213> Brevibacillus laterosporus
<400> 34
   atgaaaatta gccctctttg ccgtagattc gcggaaattc ttaacgccaa gcccacagtc 60
   gtgaatggtg tctgtactgc ccagaccctc cgtaccaaca taaaacctac tatcttaggc 120
   agaaggtcga aatctccctt aaccatccca caatttcatt cttttgaaag cattaagaag 180
   aatggtaggg ctttgtgtct tggtgagacg gtcattctca ctgaggagat caatccgtat 240
   atttcaagac ttcgagaaca tggcataaag gtaactgccc ttcataacca ctggctattt 300
   accaacccta atctatggta tatccactat gaaaagattg agaaaccttt ggtcttcgct 360
   aaaagtgtac gtgatgccct gaaagtgtta acaaaagaaa tcgtacgccc gtgtagatag 420
<210> 35
   <211> 420
   <212> DNA
   <213> Brevibacillus laterosporus
<400> 35
   ctattcacac gggcgtacga tttctttagt taacactttc agggcatcac gtacactttt      60
   agcgaagacc aaaggttcct caagcttttc atagtggata taccatagat tagggtcggt     120
   aaatagccag tggttatgaa gggcagttac ctttatgcca tgttctcgaa gtcttgaaat     180
   atacggattg atctcctcag tgagaatgac tgtctcacca agacacaaag ccctaccttt     240
   cttcttactg ttttcaaaag aatgaaattg tgggatggtt aagggagatt tcgaccttct     300
   gcctaagata gtaggtttta tgttggtacg aagggtctgg gcagtacaga caccattaac     360
   aactgtgggc tcggcgttaa gaattttcgc gaatctacgg caaagagggc taattttcat     420
<210> 36
   <211> 420
   <212> DNA
   <213> Brevibacillus laterosporus
<400> 36
   atgaaaatta gccctctttg ccgtagattc gcgaaaattc ttaacgccga gcccacagtt      60
   gttaatggtg tctgtactgc ccagaccctt cgtaccaaca taaaacctac tatcttaggc     120
   agaaggtcga aatctccctt aaccatccca caatttcatt cttttgaaaa cagtaagaag     180
   aaaggtaggg ctttgtgtct tggtgagaca gtcattctca ctgaggagat caatccgtat     240
   atttcaagac ttcgagaaca tggcataaag gtaactgccc ttcataacca ctggctattt     300
   accgacccta atctatggta tatccactat gaaaagcttg aggaaccttt ggtcttcgct     360
   aaaagtgtac gtgatgccct gaaagtgtta actaaagaaa tcgtacgccc gtgtgaatag     420
<210> 37
   <211> 420
   <212> DNA
   <213> Brevibacillus laterosporus
<400> 37
   ctatttacac gggcgtacga tttcttttgt taacactttc agggcatcac gtacactttt      60
   agcgaagacc aaaggtttct caatcttttc atagtggata taccatagat tagggttggt     120
   aaatagccag tggttatgaa gggcagttac ctttatgcca tgttctcgaa gtcttgaaat     180
   atacggattg atctcattag tgagaatgac cgtctcacca agacacaaag ccctaccatt     240
   cttcttaatg ctttcaaaag aatgaaattg tgggatggtt aagggagatt tcgaccttct     300
   gcctaagata gtaggtttta tgttggtacg gagggtctgg gcagtacaga caccattcac     360
   gactgtgggc ttggcgttaa gaatttccgc gaatctacgg caaagagggc taattttcat     420
<210> 38
   <211> 420
   <212> DNA
   <213> Brevibacillus laterosporus
<400> 38
   atgaaaatta gccctctttg ccgtagattc gcggaaattc ttaacgccaa gcccacagtc      60
   gtgaatggtg tctgtactgc ccagaccctc cgtaccaaca taaaacctac tatcttaggc     120
   agaaggtcga aatctccctt aaccatccca caatttcatt cttttgaaag cattaagaag     180
   aatggtaggg ctttgtgtct tggtgagacg gtcattctca ctaatgagat caatccgtat     240
   atttcaagac ttcgagaaca tggcataaag gtaactgccc ttcataacca ctggctattt     300
   accaacccta atctatggta tatccactat gaaaagattg agaaaccttt ggtcttcgct     360
   aaaagtgtac gtgatgccct gaaagtgtta acaaaagaaa tcgtacgccc gtgtaaatag     420
<210> 39
   <211> 259
   <212> PRT
   <213> Brevibacillus laterosporus
<400> 39
   Met Lys Arg Leu Lys Lys Val Lys Ser Lys Ile Val Ile Ser Val Leu
   1 5 10 15
   Ala Val Gly Leu Ile Gly Ser Leu Gly Thr Ala Phe Ala Ala Val Asp
   20 25 30
   Val Gly Gly Gln Leu Lys Asn Trp Tyr Asp Lys Gln Phe Ala Ala Ser
   35 40 45
   Ser Ala Ser Ile Thr Ala Glu Ala Leu Lys Tyr Gly Thr Glu Lys Lys
   50 55 60
   Ala Ala Thr Glu Ala Glu Leu Asn Lys Ile Lys Asn Gln Val Ala Asp
   65 70 75 80
   Arg Val Lys Gln Ala Gly Ser Glu Glu Ala Asn Arg Ala Val Ala Ala
   85 90 95
   Val Thr Ala Ala Lys Asn Asp Tyr Leu Asn Ala Leu Arg Gly Lys Thr
   100 105 110
   Asn Glu Ile Lys Gly Asn Met Gln Asn Gln Tyr Asn Gln Lys Val Glu
   115 120 125
   Gly Leu Lys Gln Glu Ile Thr Lys Ala Ala Asn Gln Thr Lys Val Asn
   130 135 140
   Ser Ile Val Asp Leu Thr Asn Lys Val Asn Gln Ala Gly Glu Leu Gly
   145 150 155 160
   Arg Gln Glu Val Lys Thr Asn Val Thr Ser Ala Gln Asp Lys Ala Lys
   165 170 175
   Gln Glu Leu Thr Ser Ala Ile Asn Asp Ala Lys Lys Thr Ile Asn Gly
   180 185 190
   Leu Ile Arg Glu Lys Glu Ala Ser Ser Lys Glu Glu Thr Lys Arg Phe
   195 200 205
   Ile Asp Glu Lys Val Ala Glu Ile Gln Lys Val Val Asp Glu Ser Ala
   210 215 220
   Lys Leu Leu Glu Lys Gln Ala Lys Asp Ser Ile Lys Asp Thr Gly Ala
   225 230 235 240
   Lys Ala Glu Lys Asp Ala Lys Asn Ser Leu Asp Gln Leu Val Ala Asp
   245 250 255
   Gln Phe Lys
<210> 40
   <211> 256
   <212> PRT
   <213> Brevibacillus laterosporus
<400> 40
   Met Lys Lys Val Lys Ser Lys Ile Val Ile Ser Val Leu Ala Val Gly
   1               5                   10                  15
   Leu Ile Gly Ser Leu Gly Thr Ala Phe Ala Ala Val Asp Val Gly Gly
               20                  25                  30
   Gln Leu Lys Asn Trp Tyr Asp Lys Gln Phe Ala Ala Ser Ser Ala Ser
           35                  40                  45
   Ile Thr Ala Glu Ala Leu Lys Tyr Gly Thr Glu Lys Lys Ala Ala Thr
       50                  55                  60
   Glu Ala Glu Leu Asn Lys Ile Lys Asn Gln Val Ala Asp Arg Val Lys
   65                  70                  75                  80
   Gln Ala Gly Ser Glu Glu Ala Asn Arg Ala Val Ala Ala Val Thr Ala
                   85                  90                  95
   Ala Lys Asn Asp Tyr Leu Asn Ala Leu Arg Gly Lys Thr Asn Glu Ile
               100                 105                 110
   Lys Gly Asn Met Gln Asn Gln Tyr Asn Gln Lys Val Glu Gly Leu Lys
           115                 120                 125
   Gln Glu Ile Thr Lys Ala Ala Asn Gln Thr Lys Val Asn Ser Ile Val
       130                 135                 140
   Asp Leu Thr Asn Lys Val Asn Gln Ala Gly Glu Leu Gly Arg Gln Glu
   145                 150                 155                 160
   Val Lys Thr Asn Val Thr Ser Ala Gln Asp Lys Ala Lys Gln Glu Leu
                   165                 170                 175
   Thr Ser Ala Ile Asn Asp Ala Lys Lys Thr Ile Asn Gly Leu Ile Arg
               180                 185                 190
   Glu Lys Glu Ala Ser Ser Lys Glu Glu Thr Lys Arg Phe Ile Asp Glu
           195                 200                 205
   Lys Val Ala Glu Ile Gln Lys Val Val Asp Glu Ser Ala Lys Leu Leu
   210 215 220
   Glu Lys Gln Ala Lys Asp Ser Ile Lys Asp Thr Gly Ala Lys Ala Glu
   225 230 235 240
   Lys Asp Ala Lys Asn Ser Leu Asp Gln Leu Val Ala Asp Gln Phe Lys
   245 250 255
<210> 41
   <211> 256
   <212> PRT
   <213> Brevibacillus laterosporus
<400> 41
   Leu Lys Lys Val Lys Ser Lys Ile Val Ile Ser Val Leu Ala Val Gly
   1               5                   10                  15
   Leu Ile Gly Ser Leu Gly Thr Ala Phe Ala Ala Val Asp Val Gly Gly
               20                  25                  30
   Gln Leu Lys Asn Trp Tyr Asp Lys Gln Phe Ala Ala Ser Ala Ala Ser
           35                  40                  45
   Ile Thr Thr Glu Thr Val Lys Tyr Gly Asn Glu Lys Lys Ala Val Thr
       50                  55                  60
   Glu Ala Glu Leu Asn Lys Ile Lys Asn Gln Val Ala Asp Arg Val Lys
   65                  70                  75                  80
   Gln Ala Gly Ser Glu Glu Ala Asn Arg Ala Val Ala Ala Val Thr Ala
                   85                  90                  95
   Ala Lys Asn Asp Tyr Leu Asn Ala Leu Arg Gly Lys Thr Asn Glu Ile
               100                 105                 110
   Lys Gly Asn Met Gln Asn Gln Tyr Asn Gln Lys Val Glu Gly Leu Lys
           115                 120                 125
   Gln Glu Ile Asn Lys Ala Ala Asn His Thr Lys Val Asn Ser Val Ile
       130                 135                 140
   Asp Leu Thr Asn Lys Val Asn His Ala Gly Glu Leu Gly Lys Gln Glu
   145                 150                 155                 160
   Val Arg Thr Asn Val Thr Ser Ala Gln Asp Lys Ala Lys Gln Glu Leu
                   165                 170                 175
   Thr Ser Ala Ile Asn Asp Ala Lys Lys Thr Ile Asn Gly Leu Ile Arg
               180                 185                 190
   Glu Lys Glu Ala Ser Ser Lys Glu Glu Thr Lys Arg Phe Ile Asp Glu
           195                 200                 205
   Lys Val Ala Glu Ile Gln Lys Val Val Asp Glu Ser Ala Lys Leu Leu
       210                 215                 220
   Glu Lys Gln Val Lys Asp Ala Ile Lys Asp Thr Gly Ala Lys Ala Glu
   225                 230                 235                 240
   Lys Asp Ala Lys Asn Ser Leu Asp Gln Leu Val Ala Asp Gln Phe Lys
                   245                 250                 255
<210> 42
   <211> 256
   <212> PRT
   <213> Brevibacillus laterosporus
<400> 42
   Leu Lys Lys Val Lys Ser Lys Ile Val Ile Gly Val Leu Ala Val Gly
   1               5                   10                  15
   Leu Ile Gly Ser Leu Gly Thr Ala Phe Ala Ala Val Asp Val Gly Gly
               20                  25                  30
   Gln Leu Lys Asn Trp Tyr Asp Lys Gln Phe Ala Ala Ser Ala Ser Ser
           35                  40                  45
   Ile Thr Lys Glu Ala Val Asn Tyr Gly Asn Glu Lys Lys Ala Ile Thr
       50                  55                  60
   Glu Gly Glu Ile Asn Lys Leu Lys Asn Gln Val Ala Asp Arg Val Lys
   65                  70                  75                  80
   Gln Ala Gly Ser Glu Glu Ala Asn Arg Ala Val Ala Ser Val Thr Ala
                   85                  90                  95
   Ala Lys Asn Asp Tyr Leu Asn Ala Leu Arg Gly Lys Thr Asn Glu Ile
               100                 105                 110
   Lys Gly Asn Met Gln Asn Gln Tyr Asn Gln Lys Val Glu Gly Leu Lys
           115                 120                 125
   Gln Glu Ile Asn Lys Ala Ala Asn His Thr Lys Val Asn Ser Ile Val
       130                 135                 140
   Asp Leu Thr Asn Lys Val Asn His Ala Gly Glu Ala Gly Lys Gln Glu
   145                 150                 155                 160
   Val Arg Thr Asn Val Thr Ser Ala Gln Asp Lys Ala Lys Gln Glu Leu
                   165                 170                 175
   Thr Ser Ala Ile Asn Asp Ala Lys Asn Thr Ile Asn Gly Leu Ile Arg
               180                 185                 190
   Glu Lys Glu Ala Ser Ser Lys Glu Glu Thr Lys Arg Phe Ile Asp Glu
   195 200 205
   Lys Val Ala Glu Ile Gln Lys Val Val Asp Glu Ser Ala Lys Leu Leu
   210 215 220
   Glu Lys Gln Val Lys Asp Ala Ile Lys Asp Thr Gly Ala Lys Ala Glu
   225 230 235 240
   Lys Asp Ala Lys Asn Ser Leu Asp Gln Leu Val Ala Asp Gln Phe Lys
   245 250 255
<210> 43
   <211> 259
   <212> PRT
   <213> Brevibacillus laterosporus
<400> 43
   Met Lys Arg Leu Lys Lys Val Lys Ser Lys Ile Val Ile Ser Val Leu
   1 5 10 15
   Ala Val Gly Leu Ile Gly Ser Leu Gly Thr Ala Phe Ala Ala Val Asp
   20 25 30
   Val Gly Gly Gln Leu Lys Asn Trp Tyr Asp Lys Gln Phe Ala Ala Ser
   35 40 45
   Ser Ala Ser Ile Thr Ala Glu Ala Leu Lys Tyr Gly Thr Glu Lys Lys
   50 55 60
   Ala Ala Thr Glu Ala Glu Leu Asn Lys Ile Lys Asn Gln Val Ala Asp
   65 70 75 80
   Arg Val Lys Gln Ala Gly Ser Glu Glu Ala Asn Arg Ala Val Ala Ala
   85 90 95
   Val Thr Ala Ala Lys Asn Asp Tyr Leu Asn Ala Leu Arg Gly Lys Thr
   100 105 110
   Asn Glu Ile Lys Gly Asn Met Gln Asn Gln Tyr Asn Gln Lys Val Glu
   115 120 125
   Gly Leu Lys Gln Glu Ile Thr Lys Ala Ala Asn Gln Thr Lys Val Asn
   130 135 140
   Ser Ile Val Asp Leu Thr Asn Lys Val Asn Gln Ala Gly Glu Leu Gly
   145 150 155 160
   Arg Gln Glu Val Lys Thr Asn Val Thr Ser Ala Gln Asp Lys Ala Lys
   165 170 175
   Gln Glu Leu Thr Ser Ala Ile Asn Asp Ala Lys Lys Thr Ile Asn Gly
   180 185 190
   Leu Ile Arg Glu Lys Glu Ala Ser Ser Lys Glu Glu Thr Lys Arg Phe
   195 200 205
   Ile Asp Glu Lys Val Ala Glu Ile Gln Lys Val Val Asp Glu Ser Ala
   210 215 220
   Lys Leu Leu Glu Lys Gln Ala Lys Asp Ser Ile Lys Asp Thr Gly Ala
   225 230 235 240
   Lys Ala Glu Lys Asp Ala Lys Asn Ser Leu Asp Gln Leu Val Ala Asp
   245 250 255
   Gln Phe Lys
<210> 44
   <211> 139
   <212> PRT
   <213> Brevibacillus laterosporus
<400> 44
   Met Lys Ile Ser Pro Leu Cys Arg Arg Phe Ala Glu Ile Leu Asn Ala
   1 5 10 15
   Lys Pro Thr Val Val Asn Gly Val Cys Thr Ala Gln Thr Leu Arg Thr
   20 25 30
   Asn Ile Lys Pro Thr Ile Leu Gly Arg Arg Ser Lys Ser Pro Leu Thr
   35 40 45
   Ile Pro Gln Phe His Ser Phe Glu Ser Ile Lys Lys Asn Gly Arg Ala
   50 55 60
   Leu Cys Leu Gly Glu Thr Val Ile Leu Thr Glu Glu Ile Asn Pro Tyr
   65 70 75 80
   Ile Ser Arg Leu Arg Glu His Gly Ile Lys Val Thr Ala Leu His Asn
   85 90 95
   His Trp Leu Phe Thr Asn Pro Asn Leu Trp Tyr Ile His Tyr Glu Lys
   100 105 110
   Ile Glu Lys Pro Leu Val Phe Ala Lys Ser Val Arg Asp Ala Leu Lys
   115 120 125
   Val Leu Thr Lys Glu Ile Val Arg Pro Cys Arg
   130 135
<210> 45
   <211> 139
   <212> PRT
   <213> Brevibacillus laterosporus
<400> 45
   Met Lys Ile Ser Pro Leu Cys Arg Arg Phe Ala Lys Ile Leu Asn Ala
   1               5                   10                  15
   Glu Pro Thr Val Val Asn Gly Val Cys Thr Ala Gln Thr Leu Arg Thr
               20                  25                  30
   Asn Ile Lys Pro Thr Ile Leu Gly Arg Arg Ser Lys Ser Pro Leu Thr
           35                  40                  45
   Ile Pro Gln Phe His Ser Phe Glu Asn Ser Lys Lys Lys Gly Arg Ala
       50                  55                  60
   Leu Cys Leu Gly Glu Thr Val Ile Leu Thr Glu Glu Ile Asn Pro Tyr
   65                  70                  75                  80
   Ile Ser Arg Leu Arg Glu His Gly Ile Lys Val Thr Ala Leu His Asn
                   85                  90                  95
   His Trp Leu Phe Thr Asp Pro Asn Leu Trp Tyr Ile His Tyr Glu Lys
               100                 105                 110
   Leu Glu Glu Pro Leu Val Phe Ala Lys Ser Val Arg Asp Ala Leu Lys
           115                 120                 125
   Val Leu Thr Lys Glu Ile Val Arg Pro Cys Glu
       130                 135
<210> 46
   <211> 139
   <212> PRT
   <213> Brevibacillus laterosporus
<400> 46
   Met Lys Ile Ser Pro Leu Cys Arg Arg Phe Ala Glu Ile Leu Asn Ala
   1               5                   10                  15
   Lys Pro Thr Val Val Asn Gly Val Cys Thr Ala Gln Thr Leu Arg Thr
               20                  25                  30
   Asn Ile Lys Pro Thr Ile Leu Gly Arg Arg Ser Lys Ser Pro Leu Thr
           35                  40                  45
   Ile Pro Gln Phe His Ser Phe Glu Ser Ile Lys Lys Asn Gly Arg Ala
       50                  55                  60
   Leu Cys Leu Gly Glu Thr Val Ile Leu Thr Asn Glu Ile Asn Pro Tyr
   65                  70                  75                  80
   Ile Ser Arg Leu Arg Glu His Gly Ile Lys Val Thr Ala Leu His Asn
                   85                  90                  95
   His Trp Leu Phe Thr Asn Pro Asn Leu Trp Tyr Ile His Tyr Glu Lys
               100                 105                 110
   Ile Glu Lys Pro Leu Val Phe Ala Lys Ser Val Arg Asp Ala Leu Lys
           115                 120                 125
   Val Leu Thr Lys Glu Ile Val Arg Pro Cys Lys
       130                 135
<210> 47
   <211> 20
   <212> DNA
   <213> Artificial sequence
<220>
   <223> Brevibacillus laterosporus 16S rRNA forward primer
<400> 47
   tgtagcggtg aaatgcgtag 20
<210> 48
   <211> 20
   <212> DNA
   <213> Artificial sequence
<220>
   <223> Brevibacillus laterosporus 16S rRNA reverse primer
<400> 48
   gcggcactaa gggtattgaa 20
<210> 49
   <211> 17
   <212> DNA
   <213> Artificial sequence
<220>
   <223> Brevibacillus laterosporus 16S rRNA forward primer
<400> 49
   tgaagcgaaa cggaaag 17
<210> 50
   <211> 20
   <212> DNA
   <213> Artificial Sequence
<220>
   <223> Brevibacillus laterosporus 16S rRNA reverse primer
<400> 50
   cgtcaaggtg ctaccttatt 20
<210> 51
   <211> 27
   <212> DNA
   <213> Artificial Sequence
<220>
   <223> Brevibacillus laterosporus 16 S rRNA forward primer
<400> 51
   agaccgggat aacataggga aacttat 27
<210> 52
   <211> 25
   <212> DNA
   <213> Artificial Sequence
<220>
   <223> Brevibacillus laterosporus 16 S rRNA reverse primer
<400> 52
   ggcatgctga tccgcgatta ctagc 25